(19)

Europäisches
Patentamt
European
Patent Office
Office européen
des brevets

(11) **EP 2 090 215 A1**

(12) **EUROPEAN PATENT APPLICATION**
published in accordance with Art. 153(4) EPC

(43) Date of publication:
**19.08.2009 Bulletin 2009/34**

(21) Application number: **07831603.1**

(22) Date of filing: **12.11.2007**

(51) Int Cl.:
*A61B 1/00* (2006.01)   *A61B 5/06* (2006.01)
*A61B 5/07* (2006.01)   *A61B 19/00* (2006.01)
*A61J 3/07* (2006.01)

(86) International application number:
**PCT/JP2007/071873**

(87) International publication number:
**WO 2008/059773 (22.05.2008 Gazette 2008/21)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.11.2006 JP 2006306821**

(71) Applicant: **Olympus Medical Systems Corp.**
**Tokyo 151-0072 (JP)**

(72) Inventors:
• **KAWANO, Hironao**
**Tokyo 151-0072 (JP)**

• **KIMURA, Atsushi**
**Tokyo 151-0072 (JP)**
• **UCHIYAMA, Akio**
**Tokyo 151-0072 (JP)**
• **CHIBA, Atsushi**
**Tokyo 151-0072 (JP)**

(74) Representative: **von Hellfeld, Axel**
**Wuesthoff & Wuesthoff**
**Schweigerstrasse 2**
**81541 München (DE)**

(54) **SYSTEM FOR DETECTING POSITION OF MEDICAL DEVICE, MEDICAL DEVICE GUIDANCE SYSTEM, POSITION DETECTION METHOD FOR MEDICAL DEVICE GUIDANCE SYSTEM, AND METHOD FOR GUIDING MEDICAL DEVICE GUIDANCE SYSTEM**

(57)   A medical device position detection system and a position detection method of a medical device guidance system, which are capable of detecting a direction of the medical device accurately, are provided. Included are a medical device (3) introduced into a subject's body; a magnetic field response part (45) that is disposed in the medical device (3), responds to a magnetic field by virtue of possessing a magnetization direction, and guides the medical device (3); a magnetic field generation part (45) forming the magnetic field within the subject's body; a direction detection magnetic field control part (49) generating a direction detection magnetic field from the magnetic field generation part (45) for detecting the direction of the medical device(3); a response detection part detecting a response of the magnetic field response part due to the direction detection magnetic field; and a direction calculating part (35) calculating the direction of the medical device (3) according to the direction of the direction detection magnetic field and a detection result of the response detection part.

FIG. 1

**Description**

Technical Field

The present invention relates to a medical device position detection system, a medical device guidance system, a position detection method of the medical device guidance system, and a guidance method of the medical device guidance system, which perform guidance of a medical device inserted into a body cavity.

Background Art

As a method of guiding a medical device such as a capsule endoscope in a body cavity, there have been developed magnetic guidance techniques in which a magnet is installed in a medical device , and the position and the direction of the medical device are controlled by applying a magnetic field to the magnet from the outside.

Disclosed as one of the above magnetic guidance techniques is a method of correcting rotation of an image of the medical device using a rotating magnetic field in the guidance system guiding the medical device (refer to Patent Citation 1, for example).

In this method, a theoretical rotation amount of the medical device is calculated from a coordinate transformation history by comparison of two obtained images. By image matching of the consecutive images, a rotation angle error between a rotation angle of the medical device and a rotation angle of the rotating magnetic field, which is caused by a body wall and a load of rotation, is calculated. By adding the theoretical rotation amount calculated in this manner and the rotation angle error, an actual rotation amount of the medical device between the two obtained images is calculated.

A system that guides the medical device using a magnetic gradient has also been developed (refer to Patent Citation 2, for example).

Patent Citation 1: Japanese Unexamined Patent Application, Publication No. 2003-299612
Patent Citation 2: Japanese Unexamined Patent Application, Publication No. 2005-103091

Disclosure of Invention

The above Patent Citation 1 has a problem that the amount of rotation error accumulates and increases over time, since the rotation amount of the medical device is obtained by accumulation. When the amount of rotation error accumulates, there arises a problem that the medical device cannot be controlled stably because of a discrepancy between the rotation angle of the medical device and the rotation angle of the rotating magnetic field.

The above Patent citation 2 has a problem that a magnetic attractive force cannot be generated efficiently because an angle difference between the direction of the magnet within the medical device and the direction of the generated magnetic field is not considered.

The present invention has been achieved for solving the above problems and has an object to provide a medical device position detection system and a position detection method of a medical device guidance system that are capable of detecting the direction of the medical device accurately, and a medical device guidance system and a guidance method of the medical device guidance system that are capable of performing stable and efficient guidance of the medical device.

For achieving the above object, the present invention provides the following solutions.

A first aspect of the present invention is a medical device position detection system including a medical device introduced into a subject's body, a magnetic field response part that is disposed in the medical device, responds to a magnetic field by virtue of possessing a magnetization direction, and guides the medical device; a magnetic field generation part forming a magnetic field within the subject's body, a direction detection magnetic field control part generating a direction detection magnetic field, from the magnetic field generation part, for detecting a direction of the medical device; a response detection part detecting the response of the magnetic field response part due to the direction detection magnetic field; and a direction calculation part calculating a direction of the medical device according to a direction of the direction detection magnetic field and a detection result of the response detection part.

According to the first aspect of the present invention, the medical device within the subject's body is located within the direction detection magnetic field formed by the magnetic field generation part, and thereby the magnetic field response part in the medical device responds to the direction detection magnetic field. This response of the magnetic field response part is detected by the response detection part. The direction of the medical device can be obtained highly accurately compared with the method of obtaining the rotation amount of the medical device in accumulation, since the direction of the medical device is calculated according to the direction of the direction detection magnetic field and the detected response of the magnetic field response part.

In the above first aspect of the invention, directions of two axes having different directions from each other among three axis directions different from one another in the medical device are preferably calculated by the response detection part, and a direction of an axis intersecting a plane formed by the two axes is preferably calculated by the direction calculation part.

With such a configuration, the directions of the two axes are calculated among the above three axis directions from the response of the magnetic field response part due to the direction detection magnetic field, and thereby it is not necessary to provide another detection part within the medical device, and the configuration thereof can thus be simplified.

In the above first aspect of the invention, the response detection part preferably includes an image acquisition part acquiring an image of inside the subject's body.

With such a configuration, the response of the medical device due to the direction detection magnetic field can be detected according to the image acquired by the image acquisition part. Thereby, it is not necessary to provide another detection part or the like within the medical device, and the medical device can thus be miniaturized.

In the above first aspect of the invention, the response detection part preferably includes a magnetic force measurement part measuring a force generated in the magnetic field response part.

With such a configuration, the response of the magnetic field response part is detected directly by the magnetic force measurement part, and thereby accuracy of the direction detection of the medical device can be improved. Furthermore, position detection calculation and image processing become unnecessary, and data processing for obtaining the position etc. of the medical device becomes easy to perform.

In the above first aspect of the invention, the direction detection magnetic field control part preferably controls the magnetic field generation part to generate a static magnetic field.

With such a configuration, it becomes easy to control the magnetic field generation part in generating the static magnetic field compared with a method of generating an alternating magnetic field from the magnetic field generation part.

In the above configuration, the direction detection magnetic field control part preferably controls the magnetic field generation part to sequentially generate a plurality of magnetic fields having different directions and intensities from one another, and the direction calculation part preferably calculates the direction of the medical device according to respective detection results of the response detection part for the plurality of magnetic fields.

With such a configuration, a plurality of sets of information necessary for the direction detection of the medical device according to the response of the magnetic field response part is acquired by changing the magnetic field direction or the magnetic field intensity of the direction detection magnetic field. Thereby, the system configuration of the medical device position detection system is simplified, while maintaining the same position detection accuracy of the medical device.

In the above first aspect of the invention, the direction detection magnetic field control part preferably controls the magnetic field generation part to generate a gradient magnetic field.

With such a configuration, the direction of the medical device is detected from the response of the magnetic field response part due to the gradient magnetic field formed as the direction detection magnetic field, and thereby the magnetic field generation part is commonly used as generation parts forming the direction detection magnetic field, which is a gradient magnetic field, and another field , which is a uniform magnetic field.

In the above first aspect of the invention, the directions of the two axes having different directions from each other among the three axis directions different from one another in the medical device are preferably calculated by the response detection part and the direction of the axis intersecting the plane formed by the two axes is preferably calculated by the direction calculation part, wherein the medical device preferably has a substantially cylindrical shape, the magnetization direction of the magnetic field response part is preferably substantially perpendicular to the center axis of the substantially cylindrical shape, and the plane formed by the two axes detected by the response detection part is preferably substantially parallel to the center axis.

With such a configuration, the direction detection magnetic field applied to the magnetic field response part rotates the magnetic field response part around the center axis of the substantially cylindrical shape. An angle formed by the magnetic field direction of the direction detection magnetic field and the magnetization direction of the magnetic field response part is calculated according to the rotation angle of the magnetic field response part detected by the response detection part, and the magnetization direction of the magnetic field response part is obtained.

Since the obtained magnetization direction is substantially perpendicular to the center axis and also intersects the plane formed by the two axes, the plane formed by the two axes becomes substantially parallel to the center axis.

In the above first aspect of the invention, the directions of the two axes having different directions from each other among the three axis directions different from one another in the medical device are preferably calculated by the response detection part, and the direction of the axis intersecting the plane formed by the two axes is preferably calculated by the direction calculation part, wherein the medical device preferably has a substantially cylindrical shape, the magnetization direction of the magnetic field response part is preferably substantially perpendicular to the center axis of the substantially cylindrical shape, and the plane formed by the two axes detected by the response detection part is preferably substantially perpendicular to the center axis.

With such a configuration, the direction detection magnetic field applied to the magnetic field response part rotates the magnetic field response part around the center axis of the substantially cylindrical shape. The plane formed by the two axes is obtained by obtaining a plane including the magnetization direction of the magnetic field response part before the rotation and the magnetization direction of the magnetic field response part after the rotation. Since the magnetization directions of the magnetic field response part before and after the rotation are substantially perpendicular to the center axis, the plane formed by the obtained two axes also becomes substantially perpendicular to the center axis. Accordingly, it is possible to obtain the center axis direction of the medical device by obtaining the plane formed by the two axes.

In the above first aspect of the invention, the response detection part preferably includes the magnetic force measurement part measuring the force generated in the magnetic field response part, wherein the magnetic force measurement part is preferably a sensor measuring at least one of pressure, distortion, and torque applied to the magnetic field response part, and the magnetic field response part is preferably fixed to the medical device via the sensor.

With such a configuration, the response of the magnetic field response part is directly detected by the magnetic force measurement part as at least one of pressure, distortion and torque.

Since the response of the magnetic field response part is transferred to the medical device via the magnetic force measurement part, which is a sensor, the magnetic field response part is used for the guidance of the medical device.

A second aspect of the present invention provides a medical device guidance system, including the medical device position detection system according to the first aspect of the present invention, and a guidance magnetic field control part generating a guidance magnetic field guiding the medical device from the magnetic field generation part, wherein the guidance magnetic field control part controls the magnetic field generation part according to the calculation result in the direction calculation part.

According to the second aspect of the present invention, the guidance magnetic field can be efficiently applied to the magnetic field response part, by adjustment of a magnetic field direction of the guidance magnetic field generated from the magnetic field generation part according to the calculation result in the medical device direction calculation part. It is possible to apply the magnetic field with respect to the direction of the magnetic field response part as intended and to control the medical device stably.

In the second aspect of the invention, the intensity of the magnetic field generated from the magnetic field generation part by the direction detection magnetic field control part is preferably lower than the intensity of the magnetic field generated from the magnetic field generation part by the guidance magnetic field control part.

With such a configuration, because the magnetic field intensity of the direction detection magnetic field is lower than that of the guidance magnetic field, it is difficult to change the position and the direction of the medical device with the direction detection magnetic field, making it difficult to influence guidance of the medical device by the guidance magnetic field.

For example, when measuring the force generated in the magnetic field response part with the magnetic force measurement part, it is possible to carry out the direction detection highly accurately without moving the medical device, by setting the intensity of the direction detection magnetic field to be not lower than the intensity that enables the magnetic force measurement part to measure the above force and also to be not higher than the intensity that can move the medical device.

A third aspect of the present invention provides a position detection method of the medical device guidance system, including the steps of: a magnetic field generation part generating a direction detection magnetic field for detecting a magnetization direction of a magnetic field response part provided in a medical device; detecting a response of the magnetic field response part; and detecting a direction of the medical device from the response of the magnetic field response part and a direction of the direction detection magnetic field.

According to the third aspect of the present invention, the medical device is located within the direction detection magnetic field formed by the magnetic field generation part, and thereby the magnetic field response part in the medical device responds to the direction detection magnetic field. This response of the magnetic field response part is detected by the response detection part. The direction of the medical device is calculated according to the direction of the direction detection magnetic field and the detected response of the magnetic field response part, and thereby the direction of the medical device can be obtained highly accurately compared with the method of obtaining the rotation amount of the medical device in accumulation.

A fourth aspect of the present invention provides a guidance method of the medical device guidance system, wherein after the step of detecting a direction in a position detection method of the medical device guidance system according to the third aspect of the present invention, the guidance method includes the step of the magnetic field generation part generating a guidance magnetic field, for guiding the medical device, according to the detected direction of the medical device.

According to the fourth aspect of the present invention, the guidance magnetic field can be efficiently applied to the magnetic field response part by adjustment of the direction of the guidance magnetic field generated from the magnetic field generation part. It is possible to apply the magnetic field with respect to the direction of the magnetic field response part as intended and to control the medical device stably.

The medical device position detection system according to the first aspect of the present invention and the position detection method of the medical device guidance system according to the third aspect calculate the direction of the medical device according to the direction of the direction detection magnetic field and the detected response of the magnetic field response part, and thereby have an advantage of being able to detect the direction of the medical device accurately.

The medical device guidance system according to the second aspect of the present invention and the guidance method of the medical device guidance system according to the fourth aspect adjust the magnetic field direction of the guidance magnetic field generated from the magnetic field generation part according to the calculation result in the medical device direction calculation part, and thereby have an advantage of being able to control the guidance of the medical device stably and efficiently.

Brief Description of Drawings

[FIG. 1] Fig. 1 is a schematic diagram illustrating, in outline, a medical device guidance system according to a first embodiment of the present invention.

[FIG. 2] Fig. 2 is a diagram illustrating coordinate axes in a capsule medical device of Fig. 1 and coordinate axes in an external device.

[FIG. 3] Fig. 3 is a schematic diagram illustrating the internal configuration of the capsule medical device of Fig. 1.

[FIG. 4] Fig. 4 is a schematic diagram illustrating the configuration of a position and orientation detection part of Fig. 1.

[FIG. 5] Fig. 5 is a diagram illustrating another drive method of the capsule medical device of Fig. 2.

[FIG. 6] Fig. 6 is a schematic diagram illustrating the configuration of an interface of Fig. 1.

[FIG. 7] Fig. 7 is an outline diagram illustrating the configuration of an operation part of Fig. 6.

[FIG. 8] Fig. 8 is an outline diagram illustrating the configuration of the operation part of Fig. 6.

[FIG. 9] Fig. 9 is a schematic diagram illustrating a display part of Fig. 6.

[FIG. 10] Fig. 10 is a diagram illustrating an image captured by a capsule medical device when a direction detection magnetic field $M_1$ having a magnetic field intensity $H_1$ is applied.

[FIG. 11] Fig. 11 is a diagram illustrating an image captured by the capsule medical device when the direction detection magnetic field $M_1$ having a magnetic field intensity $H_2$ is applied.

[FIG. 12] Fig. 12 is a diagram showing a relationship among a magnetic field of a permanent magnet, a direction detection magnetic field, and a torque applied to the permanent magnet.

[FIG. 13] Fig. 13 is a diagram illustrating a method of detecting an angle shift between the magnetic field direction of a direction detection magnetic field and the magnetization direction of the permanent magnet in a first modification of the first embodiment of the present invention.

[FIG. 14] Fig. 14 is a diagram illustrating the method of detecting the angle shift between the magnetic field direction of the direction detection magnetic field and the magnetization direction of the permanent magnet in the first modification of the first embodiment in the present invention.

[FIG. 15] Fig. 15 is a perspective view illustrating a rotating magnetic field formed for obtaining the magnetization direction of a permanent magnet in a second modification of the first embodiment of the present invention.

[FIG. 16] Fig. 16 is a perspective view illustrating a rotating magnetic field $M_{R2}$ formed for obtaining the magnetization direction of a permanent magnet in the second modification of the first embodiment of the present invention.

[FIG. 17] Fig. 17 is a top view along a Z-axis showing the rotating magnetic field of Fig. 16.

[FIG. 18] Fig. 18 is a diagram showing a relationship among the direction of a magnetic field generated by a rotating magnetic field $M_{R1}$, an angle difference $\theta$ thereof from an X-Y plane, an angle difference $\varphi$ of a permanent magnet from the X-Y plane, and an angle difference $\Delta\theta$ between the generated magnetic field direction and the magnetization direction of the permanent magnet.

[FIG. 19] Fig. 19 is a diagram showing a relationship among the direction of a magnetic field generated by a rotating magnetic field $M_{R2}$, an angle difference $\theta$ thereof from an X-Y plane, an angle difference $\varphi$ of a permanent magnet from the X-Y plane, and an angle difference $\Delta\theta$ between the generated magnetic field direction and the magnetization direction of the permanent magnet.

[FIG. 20] Fig. 20 is a diagram comparing images captured by an image sensor in a capsule medical device when directions of magnetic fields generated by rotating magnetic fields $M_{R1}$ and $M_{R2}$ are the same, where FIG. 20(a) is an image of the rotating field $M_{R1}$, and FIG. 20(b) is an image of the rotating field $M_{R2}$.

[FIG. 21] Fig. 21 is a cross-sectional view illustrating the configuration of a permanent magnet and a surrounding part thereof in a capsule medical device in a third modification of the first embodiment of the present invention.

[FIG. 22] Fig. 22 is a diagram illustrating application of a gradient magnetic field to the capsule medical device of Fig. 21.

[FIG. 23] Fig. 23 is a diagram illustrating application of another gradient magnetic field to the capsule medical device of Fig. 21.

[FIG. 24] Fig. 24 is a cross-sectional view illustrating the configuration of a permanent magnet and a surrounding part thereof in a capsule medical device in a fourth modification of the first embodiment of the present invention.

[FIG. 25] Fig. 25 is a diagram illustrating a case where, instead of a capsule medical device, an endoscope is used as a medical device applied to a medical device guidance system of the present invention.

[FIG. 26] Fig. 26 is a schematic diagram illustrating, in outline, a medical device guidance system according to a second embodiment of the present invention.

[FIG. 27] Fig. 27 is a schematic diagram illustrating a part different from that in the first embodiment in the medical device guidance system of Fig. 26.

[FIG. 28] Fig. 28 is a schematic diagram illustrating a relationship among the magnetic field direction of a static magnetic field applied to the capsule medical device of Fig. 26, the magnetization direction of a permanent magnet, and an f-axis direction of the capsule medical device.

[FIG. 29] Fig. 29 is a diagram illustrating a relationship between the magnetic field direction of the static magnetic field applied to the capsule medical device and the magnetization direction of the permanent magnet, when viewed in the f-axis direction of Fig. 28.

[FIG. 30] Fig. 30 is a schematic diagram illustrating the magnetic field direction of a magnetic field generated for direction detection in the first modification of the second embodiment of the present invention.

[FIG. 31] Fig. 31 is a diagram illustrating an image captured before a plurality of direction detection magnetic fields $M_1$ is generated.

[FIG. 32] Fig. 32 is a diagram illustrating an image captured after the plurality of direction detection magnetic fields $M_1$ is generated.

[FIG. 33] Fig. 33 is a diagram illustrating a relationship between magnetization directions of a permanent magnet before and after generation of a magnetic field for position detection and the magnetic field direction of the magnetic field for position detection in a second modification of the second embodiment of the present invention.

[FIG. 34] Fig. 34 is a vector diagram illustrating a method of calculating a rotation plane and a turning plane in the capsule medical device of Fig. 33.

[FIG. 35] Fig. 35 is a diagram illustrating the configuration of a permanent magnet and a surrounding part thereof in the capsule medical device and the magnetic field direction of a direction detection magnetic field applied to the permanent magnet in a third modification of the second embodiment of the present invention.

[FIG. 36] Fig. 36 is a perspective view illustrating the magnetization direction of the direction detection magnetic field of Fig. 35.

[FIG. 37] Fig. 37 is a diagram illustrating an angle formed by the magnetization direction of the direction detection magnetic field and the Z-axis in Fig. 38.

[FIG. 38] Fig. 38 is a diagram illustrating an angle formed by the magnetization direction of the direction detection magnetic field and the Z-axis in Fig. 36.

[FIG. 39] Fig. 39 is a schematic diagram illustrating another example of the capsule medical device applied to the second embodiment of the present invention.

[FIG. 40] Fig. 40 is a front view illustrating the configuration of the capsule medical device of Fig. 39.

[FIG. 41] Fig. 41 is a vertical cross-sectional view illustrating the configuration of a permanent magnet and a surrounding part thereof in a capsule medical device of a third embodiment of the present invention.

[FIG. 42] Fig. 42 is a front view illustrating the configuration of the permanent magnet and the surrounding part thereof in the capsule medical device of Fig. 41.

[FIG. 43] Fig. 43 is a vector diagram illustrating a position detection magnetic field applied to the capsule medical device of Fig. 41.

[FIG. 44] Fig. 44 is a vertical cross-sectional view illustrating the configuration of a permanent magnet and a surrounding part thereof in a capsule medical device of a fourth embodiment of the present invention.

[FIG. 45] Fig. 45 is a front view illustrating the configuration of the permanent magnet and the surrounding part thereof in the capsule medical device of Fig. 44.

[FIG. 46] Fig. 46 is a vector diagram illustrating a position detection magnetic field applied to the capsule medical device of Fig. 44.

[FIG. 47] Fig. 47 is a vector diagram illustrating another application example of the position detection magnetic field applied to the capsule medical device of Fig. 44.

[FIG. 48] Fig. 48 is a diagram illustrating a method of determining an f-axis direction in the capsule medical device of Fig. 47.

[FIG. 49] Fig. 49 is a diagram illustrating a case where, instead of a capsule medical device, an endoscope device is used as a medical device applied to a medical device guidance system in the fourth embodiment of the present invention.

[FIG. 50] Fig. 50 is a front view illustrating the configuration of the endoscope device of Fig. 49.

Description of Reference Signs:

1, 301, 501, 601: Medical device guidance system
3, 103, 203, 303, 403, 503, 603: Capsule medical device (Medical device)
9: Imaging part (Image acquisition part, Response detection part)
21, 321, 521, 721: Permanent magnet (Magnetic field response part)
35: Direction calculation part
45: Magnetic field generation part
49: Direction detection magnetic field control part
122: Pressure sensor (Magnetic force measurement part)
222, 422, 522R, 522F, 622, 722: Force sensor (Magnetic force measurement part)
303, 703: Endoscope device (Medical device)

Best Mode for Carrying Out the Invention

[First embodiment]

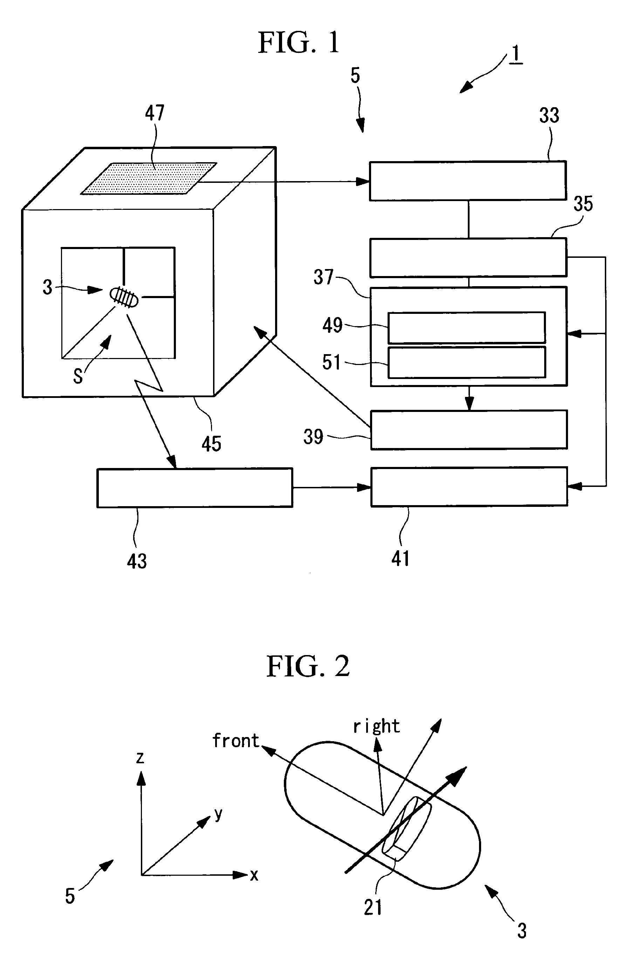

Hereinafter, a medical device guidance system according to a first embodiment of the present invention will be described with reference to Fig. 1 to Fig. 12.
Fig. 1 shows a schematic diagram illustrating, in outline, the medical device guidance system according to the present embodiment.
As shown in Fig. 1, a medical device guidance system 1 is provided with a capsule medical device (medical device) 3 that is introduced into a body cavity of a subject's body, and an external device that detects the position and direction of the capsule medical device 3 and also guides the capsule medical device 3.
Fig. 2 shows a diagram illustrating coordinate axes in the capsule medical device of Fig. 1 and coordinate axes in the external device.
The capsule medical device 3 has right-handed coordinate axes composed of an up-axis (hereinafter, denoted by u-axis), a right-axis (hereinafter, denoted by r-axis), and a front-axis (hereinafter, denoted by f-axis), as shown in Fig. 2. Specifically, the u-axis extends in the magnetization direction of a permanent magnet 21, the r-axis extends in the radial direction of the capsule medical device 3, and the f-axis extends in the longitudinal axis direction of the capsule medical device 3. On the other hand, the external device 5 has a right-handed coordinate system composed of an x-axis, a y-axis, and a z-axis.

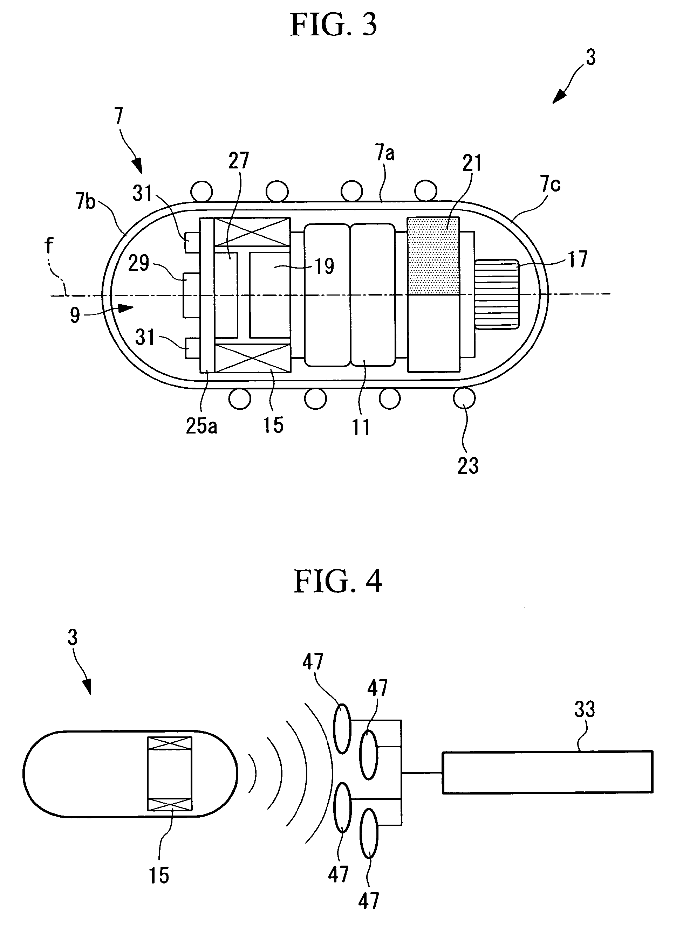

Fig. 3 shows a schematic diagram illustrating the internal configuration of the capsule medical device of Fig. 1.
As shown in Fig. 3, the capsule medical device 3 is provided with an exterior 7 containing various kinds of equipment therein, an imaging part (image acquisition part, response detection part) 9 acquiring a body cavity inside image of the subject's body, a power supply part 11 supplying power to the various kinds of equipment inside the exterior 7, an oscillation coil 15 generating an oscillating magnetic field, a wireless transmitter 17 transmitting image data or the like outside the body, a control part 19 controlling the power supply part 11, the imaging part 9, the oscillation coil 15, and the wireless transmitter 17, and a permanent magnet (magnetic field response part) 21 responding to a magnetic field.
The exterior 7 is formed of a cylindrical capsule main body 7a that transmits infra-red light and has a center axis at the f-axis of the capsule medical device 3, a hemispherical transparent front-end part 7b that covers a front end of the capsule main body 7a, and a hemispherical rear-end part 7c that covers a rear end of the capsule main body 7a, forming a hermetically sealed capsule container having a water-tight structure.
The outer peripheral surface of the capsule main body 7a in the exterior 7 is provided with a spiral part 23 in which a wire having a circular cross-section is spirally wound around the f-axis. Thereby, the capsule medical device is rotated around the f-axis to go forward or backward.
The imaging part 9 acquires an image by imaging the body cavity inside the subject. The imaging part 9 is provided with an image sensor 27, which is an imaging element, disposed in the plane of a substrate 25a, which is disposed substantially perpendicular to the f-axis direction, on the side of the rear-end part 7c, a lens group 29 forming an image of the body cavity inside surface in the subject on the image sensor 27, and an LED (Light Emitting Diode) 31 illuminating the body cavity inside surface.
The image sensor 27 converts light focused through the front-end part 7b and the lens group 29 into an electrical signal (image signal) and outputs it to the control part 19. For this image sensor 27, it is possible to use an imaging element such as a CMOS (Complementary Metal Oxide Semiconductor) device and a CCD (Charge Coupled Device), for example.

A plurality of the LEDs 31 is disposed in the circumferential direction around the f-axis with gaps therebetween.

The oscillation coil 15 is used for generating the oscillating magnetic field and is wound in a cylindrical shape to be disposed within the capsule main body 7a of the exterior 7 in the radial direction.

That is, the center axis line of the oscillation coil 15 is disposed substantially parallel to the f-axis direction, and the opening direction of the oscillation coil 15 is disposed in a direction perpendicular to the magnetization direction of the permanent magnet 21.

The control part 19 is electrically connected to the power supply part 11, the oscillation coil 15, the image sensor 27, and the LEDs 31. The control part 19 transmits the image signal acquired by the image sensor 27 from the wireless transmitter 17 and also controls the ON and OFF state of the oscillation coil 15, the image sensor 27, and the LEDs 31.

The permanent magnet 21 generates a driving force according to a direction detection magnetic field $M_1$ and a guidance magnetic field $M_2$ applied by the external device 5. The permanent magnet 21 is disposed on the rear-end part 7c side of the wireless transmitter 17. The permanent magnet 21 is disposed or magnetized so as to have a magnetization direction (magnetic poles) in a direction perpendicular to the f-axis direction (e.g., vertical direction in the drawing).

The external device 5 is provided with a position and orientation detection part 33, a direction calculation part 35, a magnetic field control part 37, a power supply 39, an interface 41, an image data receiving part 43, and a magnetic field generation part 45, as shown in Fig. 1.

Fig. 4 is a schematic diagram illustrating the configuration of the position and orientation detection part of Fig. 1. The position and orientation detection part 33 detects five degrees of freedom of coordinate values (position) of the capsule medical device 3 in the coordinate system in the external device 5 and the direction of the f-axis in the capsule medical device 3, that is, rotation phases of the capsule medical device 3 around the u-axis and the r-axis. A method of calculating these coordinate values and rotation phases can be realized by a publicly known calculation method and is not limited particularly. The position and orientation detection part 33 is provided with a plurality of detection coils 47 and receives detection signals from the detection coils 47, as shown in Fig. 4.

The detection coils 47 detect the oscillating magnetic field excited by the oscillation coil 15 in the capsule medical device 3 and are disposed around a working region of the capsule medical device 3.

The direction calculation part 35 calculates one degree of freedom of a rotation phase of the capsule medical device 3 in the u-axis direction and the r-axis direction, that is, the rotation phase of the capsule medical device 3 around the f-axis (direction of the medical device), as shown in Fig. 1. The method of calculating the rotation phase will be described in detail hereinafter. The rotation phase of the capsule medical device 3 around the f-axis is input into a data processing part 53 in the interface 41 from the direction calculation part 35, as shown in Fig. 1.

The magnetic field control part 37 outputs a magnetic field formation signal forming the direction detection magnetic field $M_1$ and the guidance magnetic field $M_2$ in the working region of the capsule medical device 3, as shown in Fig. 1. The direction detection magnetic field $M_1$ is a magnetic field used for detecting the phase of the capsule medical device 3 around the f-axis, that is, the magnetization direction of the permanent magnet 21. The guidance magnetic field $M_2$ is a magnetic field guiding the capsule medical device 3 and is also a rotating magnetic field controlling the f-axis direction of the capsule medical device 3 and also driving the capsule medical device 3 to rotate around the f-axis.

The magnetic field control part 37 is provided with a direction detection magnetic field control part 49 generating the magnetic field formation signal controlling the magnetic field direction and the magnetic field intensity of the direction detection magnetic field $M_1$ and a guidance magnetic field control part 51 outputting a magnetic field formation signal controlling the magnetic field direction and the magnetic field intensity of the guidance magnetic field $M_2$.

The direction detection magnetic field control part 49 outputs the magnetic field formation signal controlling the magnetic field generated by the magnetic field generation part 45 when the direction of the capsule medical device 3 is to be detected. On the other hand, the guidance magnetic field control part 51 outputs the magnetic field control signal controlling the magnetic field generated by the magnetic field generation part 45 when the capsule medical device 3 is to be guided.

The magnetic field control part 37 receives the rotation phase of the capsule medical device 3 around the f-axis from the direction calculation part 35 and receives operation information input by an operator from the data processing part 53 in the interface 41.

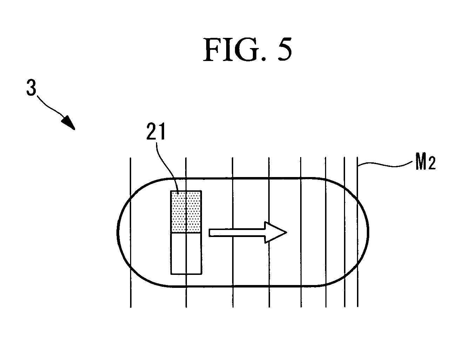

Fig. 5 is a diagram illustrating another drive method of the capsule medical device in Fig. 2.

Note that the guidance magnetic field $M_2$ may be the rotating magnetic field and the capsule medical device 3 may be driven by the rotating magnetic field as described above, or the guidance magnetic field $M_2$ may be a gradient magnetic field, which is a static magnetic field having a magnetic gradient, and the capsule medical device 3 may be driven by the gradient magnetic field, as shown in Fig. 5; the drive method is not particularly limited. Fig. 5 shows formation of a guidance magnetic field that has a sharper gradient toward the right side and shows a state where a magnetic attractive force is applied for driving the capsule medical device 3 to move it toward the right side.

By generating the gradient magnetic field from the magnetic field generation part 45 in this manner, it becomes easy to control the magnetic generation part 45 compared with the method of generating the rotating magnetic field, which is an alternating magnetic field.

The power supply 39 outputs alternating electric power for generating the direction detection magnetic field $M_1$ and the guidance magnetic field $M_2$ from the magnetic field generation part 45 according to the control signal from the magnetic field control part 37, as shown in Fig. 1. The power supply 39 receives a control signal from the magnetic field control part 37 and supplies the alternating electric power to the magnetic field generation part 45, as shown in Fig. 1.

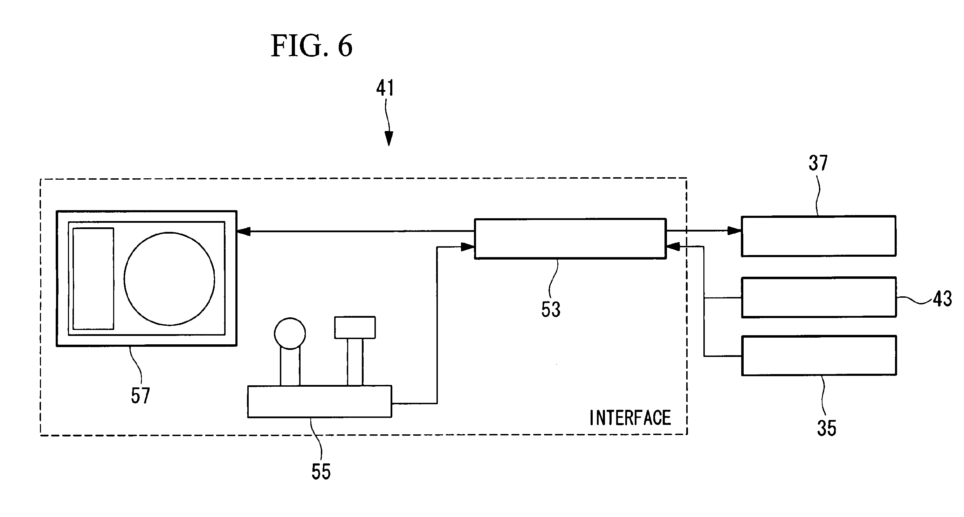

Fig. 6 is a schematic diagram illustrating the configuration of the interface of Fig. 1.

The interface 41 receives a manipulated variable of the capsule medical device 3 from the operator and also displays the image acquired by the capsule medical device 3. The interface 41 is provided with the data processing part 53, an operation part 55, and a display part 57, as shown in Fig. 6.

The data processing part 53 calculates image data to be displayed on the display part 57 and also calculates the operation information to be input to the magnetic field control part 37. Specifically, the data processing part 53 converts image data that is input from the image data receiving part 43 and that rotates along with the rotation of the capsule medical device 3 into static image data according to the rotation phase of the capsule medical device 3 around the f-axis.

The data processing part converts the operation information that is input from the operation part 55 and is based on the coordinate system of the capsule medical device (coordinate system composed of the u-axis, r-axis, and f-axis) into the coordinate system of the external device 5 (coordinate system composed of the x-axis, y-axis, and z-axis), according to the rotation phase of the capsule medical device 3 around the f-axis.

The data processing part 53 receives the image data from the image data receiving part 43 and also receives the rotation phase of the capsule medical device 3 around the f-axis from the direction calculation part 35, and the data processing part 53 outputs the static image data to the display part 57. The data processing part 53 receives the operation information based on the coordinate system of the capsule medical device 3 regarding a travel direction and a travel speed of the capsule medical device 3 from the operation part 55, converts the operation information into operation information based on the coordinate system of the external device 5, and outputs the converted operation information to the magnetic field control part 37.

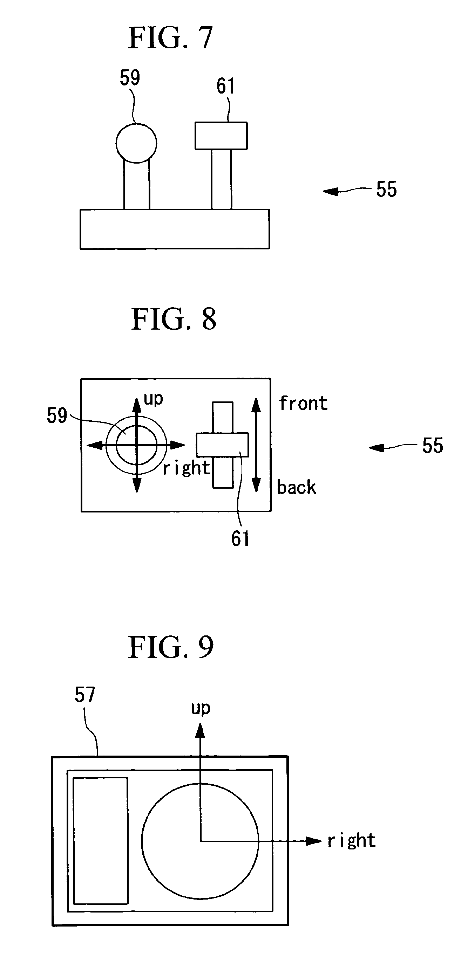

Fig. 7 and Fig. 8 are schematic diagrams illustrating the configuration of the operation part of Fig. 6.

The operation part 55 receives the travel direction and the travel speed of the capsule medical device 3 (operation information) from the operator. The operation part 55 is provided with a direction control part 59 to which the travel direction of the capsule medical device 3 is input and an accelerator 61 to which the speed of travel, including forward travel and reverse travel, of the capsule medical device 3 is input, as shown in Fig. 7 and Fig. 8.

The direction control part 59 is a rod-shaped member provided so as to be tilted along two directions perpendicular to each other. When the direction control part 59 is tilted to the up side in the drawing, the capsule medical device 3 is controlled to change the direction thereof to the positive direction on the up-axis, and when the direction control part 59 is tilted in the reverse direction, the capsule medical device 3 is controlled to change the direction thereof to the negative direction on the up-axis. Similarly, when the direction control part 59 is tilted to the right side in the drawing, the capsule medical device 3 is controlled to change the direction thereof to the positive direction on the right-axis, and when the direction control part 59 is tilted in the reverse direction, the capsule medical device 3 is controlled to change the direction thereof to the negative direction on the right-axis.

The accelerator 61 is a rod-shaped member provided to be tilted along one direction. When the accelerator is tilted to the front-side in the drawing, the capsule medical device 3 is controlled to travel forward, that is, in the positive direction on the f-axis, and when the accelerator is tilted to the back-side, the capsule medical device 3 is controlled to travel backward, that is, in the negative direction on the f-axis. Regardless of the tilted direction of the accelerator 61, the travel speed of the capsule medical device 3 is controlled according to the tilted angle of the accelerator 61.

Fig. 9 is a schematic diagram illustrating the display part of Fig. 6.

The display part 57 displays the image data captured by the capsule medical device 3. The display part 57 receives the static image data from the data processing part 53 and displays the static image as shown in Fig. 6. Specifically, the display part 57 displays the static image such that the up-axis and the right-axis on the display part 57 correspond to the u-axis and the r-axis of the capsule medical device 3, respectively, as shown in Fig. 9.

The image data receiving part 43 receives the image data transmitted from the wireless transmitter 17 in the capsule medical device 3, as shown in Fig. 1. The image data received by the image data receiving part 43 is input to the data processing part 53 in the interface 41, as shown in Fig. 6.

The magnetic field generation part 45 forms the direction detection magnetic field $M_1$ and the guidance magnetic field $M_2$ using the alternating electric power supplied from the power supply 39. The magnetic field generation part 45 can be configured with publicly known coils, such as Helmholtz coils, and is not particularly limited.

While the present embodiment is explained in the case where the same coil is used for forming the direction detection magnetic field $M_1$ and the guidance magnetic field $M_2$, a coil forming the direction detection magnetic field $M_1$ and a coil forming the guidance magnetic field $M_2$ may be provided separately; it is not limited particularly.

Next, the operation of the capsule medical device in the medical device guidance system 1 having the configuration described above will be described.

First, an outline of a guiding method and an image acquisition method of the capsule medical device 3 will be described, and then a method of detecting the rotation phase of the capsule medical device 3 around the f-axis and a method of guiding the capsule medical device 3 using the detected rotation phase, which are features of the present embodiment, will be described.

First, a subject is disposed in a space S where the guidance magnetic field $M_2$ is formed by the magnetic field generation part 45, as shown in Fig. 1.

Next, the power supply of the capsule medical device 3 is turned on, and the capsule medical device 3 is put into the body cavity of the subject from the mouth or the anus. Also in the external device 5, electric power starts to be supplied to the position and orientation detection part 33 and so forth.

The capsule medical device 3 input into the body cavity starts operating the imaging part 9 after a predetermined time and makes the image sensor 27 acquire an image of the body cavity inside surface illuminated by the illumination light from the LED 31. The acquired image data is transferred to the wireless transmitter 17 via the control part 19 and is transmitted to the image data receiving part 43 via the wireless transmitter 17.

The image data is input to the data processing part 53 in the interface 41 from the image data receiving part 43. The data processing part 53 converts the image data, which is rotating along with the rotation of the capsule medical device 3, into a static image data according to the rotation phase of the capsule medical device 3 around the f-axis, which is calculated by the direction calculation part 35. The converted image data is output to the display part 57, and the display part 57 displays the image of the body cavity inside surface.

An operator, after having confirmed the image of the body cavity inside surface displayed on the display part 57, inputs the operation information, such as the travel direction and the travel speed, of the capsule medical device 3 into the data processing part 53, by operating the operation part 55 in the interface 41. The operation information input from the operation part is operation information based on the coordinate system of the capsule medical device, and therefore, the data processing part converts the operation information into operation information based on the coordinate system of the external device 5 using the rotation phase of the capsule medical device 3 around the f-axis, which is calculated by the direction calculation part 35.

The converted operation information is input into the magnetic field control part 37. The magnetic field control part 37 determines the rotation axis direction, rotation direction, rotation speed and so forth for the guidance magnetic field $M_2$ of the rotating magnetic field in the guidance magnetic field control part 51 and outputs the magnetic field formation signal forming the guidance magnetic field $M_2$ to the power supply 39. The power supply 39 supplies the alternating electric power generated according to the input magnetic field formation signal to the magnetic field generation part 45. Thereby, the magnetic field generation part is magnetized to form the desired guidance magnetic field $M_2$. The rotation axis direction of the guidance magnetic field $M_2$ controls the direction of the capsule medical device 3 (f-axis direction) and the travel direction thereof, the rotation direction controls forward travel and backward travel of the capsule medical device 3, and the rotation speed controls the travel speed of the capsule medical device 3.

Next, the method of detecting the rotation phase of the capsule medical device 3 around the f-axis, that is, an angle shift between the magnetic field direction of the direction detection magnetic field and the magnetization direction of the permanent magnet, will be described.

First, the direction detection magnetic field control part 49 in the magnetic field control part 37 outputs to the magnetic field generation part 45 the magnetic field formation signal generating the direction detection magnetic field $M_1$, which has a magnetic field intensity of $H_1$ and any magnetic field direction in a plane (u-r plane) perpendicular to the f-axis direction of the capsule medical device 3, which is calculated by the position and orientation detection part 33. Thereby, the magnetic field generation part 45 generates the direction detection magnetic field $M_1$.

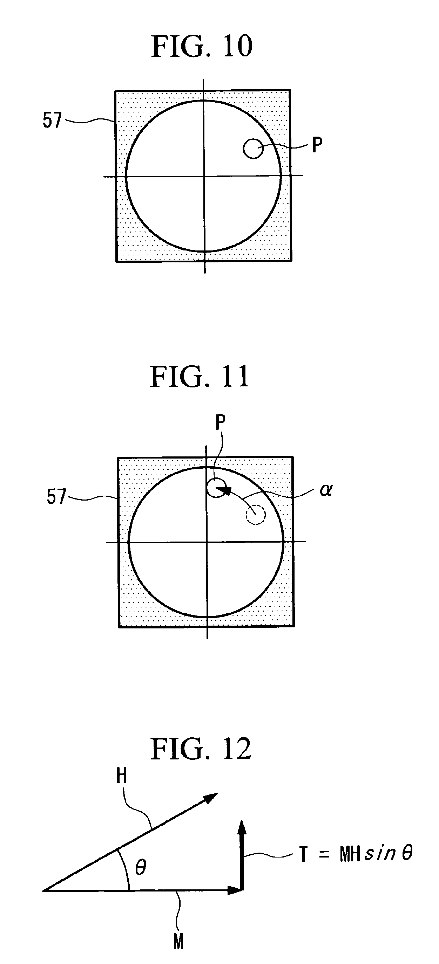

Fig. 10 is a diagram illustrating an image captured by the capsule medical device when the direction detection magnetic field $M_1$ having the magnetic field intensity of $H_1$ is applied.

The image sensor 27 acquires an image of the body cavity inside wall, as shown in Fig. 10, when the direction detection magnetic field $M_1$ is applied to the permanent magnet 21 in the capsule medical device 3. Here, a detection pattern P is set for detecting the angle shift between the magnetic field direction of the direction detection magnetic field $M_1$ and the magnetization direction of the permanent magnet 21.

Then, the direction detection magnetic field control part 49 outputs to the magnetic field generation part 45 a magnetic field formation signal that increases the magnetic field intensity of the direction detection magnetic field $M_1$ to $H_2$ while keeping the same magnetic field direction. Thereby, the magnetic field generation part 45 generates a direction detection magnetic field $M_1$ having a magnetic field intensity of $H_2$ (detection magnetic field generation step).

When the magnetic field intensity of the direction detection magnetic field $M_1$ is increased, the torque T applied to the permanent magnet 21 increases, and the capsule medical device 3 rotates around the f-axis.

Fig. 11 is a diagram illustrating an image captured by the capsule medical device 3 when the direction detection magnetic field $M_1$ having the magnetic field intensity $H_2$ is applied.

After the direction detection magnetic field $M_1$ having the magnetic field intensity $H_2$ is applied to the capsule medical device 3 in this manner, the image sensor 27 acquires an image of the body cavity inside wall, as shown in Fig. 11. The captured detection pattern P in the image at this time rotates by an angle $\alpha$ compared to the image shown in Fig. 10. The direction calculation part 35 detects the rotation angle $\alpha$ of the detection pattern P by comparing the images shown in Fig. 10 and Fig. 11, and thereby obtains the rotation angle $\alpha$ of the capsule medical device 3 around the f-axis (response detection step). It is possible to obtain $\alpha$ more accurately by setting the plurality of detection patterns P.

Fig. 12 is a diagram showing the relationship among the magnetic field of the permanent magnet, the direction detection magnetic field, and the torque applied to the permanent magnet.

From the relational expressions of the torques applied to the permanent magnet 21 before and after the change of the magnetic field intensity in the direction detection magnetic field $M_1$ and a relational expression between the rotation angle $\alpha$ and a change in an angle $\theta$ formed by the magnetic field direction of the direction detection magnetic field $M_1$ and the magnetization direction of the permanent magnet 21, the direction calculation part 35 calculates the formed angle $\theta$ (angle shift) (direction detection step).

Here, the torque applied to the permanent magnet 21 having a magnetic field intensity M, by the direction detection magnetic field $M_1$ having the magnetic field intensity H, can be obtained by the following formula (1) using the diagram shown in Fig. 12.

$$T = MH \cdot \sin \theta \qquad \cdots (1)$$

Since the torque applied to the permanent magnet 21 does not change between before and after the change in the magnetic field intensity of the direction detection magnetic field $M_1$, the following formula (2) is introduced.

$$MH_1 \cdot \sin \theta_1 = MH_2 \cdot \sin \theta_2 \qquad \cdots (2)$$

Here, $\theta_1$ is the angle formed by the magnetic field direction of the direction detection magnetic field $M_1$ and the magnetization direction of the permanent magnet 21 when the direction detection magnetic field $M_1$ having the magnetic field intensity $H_1$ is formed, and $\theta_2$ is the angle formed by the magnetic field direction of the direction detection magnetic field $M_1$ and the magnetization direction of the permanent magnet 21 when the direction detection magnetic field $M_1$ having the magnetic field intensity $H_2$ is formed.

The rotation angle $\alpha$ is expressed by the following formula (3) using the formed angles $\theta_1$ and $\theta_2$.

$$\theta_1 - \theta_2 = \alpha \qquad \cdots (3)$$

The direction calculation part 35 calculates the angles $\theta_1$ and $\theta_2$ formed by the magnetic field direction of the direction detection magnetic field $M_1$ and the magnetization direction of the permanent magnet 21, from the above formula (2) and formula (3).

The magnetization direction of the permanent magnet 21, that is, the rotation phase (direction or orientation) of the capsule medical device 3 around the f-axis, is obtained from the formed angles $\theta_1$ and $\theta_2$ calculated in this manner and the magnetic field direction of the direction detection magnetic field $M_1$.

The rotation phase of the capsule medical device 3 around the f-axis obtained by the direction calculation part 35 is input into the guidance magnetic field control part in the magnetic field control part 37. The guidance magnetic field control part determines the magnetic field direction of the guidance magnetic field $M_2$ according to the rotation phase of the capsule medical device 3 around the f-axis and generates the guidance magnetic field $M_2$ from the magnetic field generation part (guidance magnetic field generation step).

The guidance and the direction detection of the capsule medical device 3 are repeated alternately, and the result of the direction detection of the capsule medical device 3 is fed back to the control of the guidance magnetic field $M_2$. It is possible to control the capsule medical device 3 more efficiently and more stably by controlling the magnetic field direction of the guidance magnetic field $M_2$ so that the above formed angle $\theta$ has an appropriate value.

With the above configuration, the direction calculation part 35 calculates the angle $\theta$ (angle difference) formed by the magnetization direction of the permanent magnet 21 and the magnetic field direction of the direction detection magnetic field $M_1$, and thereby it is possible to carry out position and orientation detection with six degrees of freedom using the position and orientation detection part, which detects five degrees of freedom among the position and orientation (six degrees of freedom) of the capsule medical device.

The formed angle θ is calculated in the direction calculation part 35 from the rotation angle α of the capsule medical device 3 (permanent magnet 21) when the magnetic field intensity of the direction detection magnetic field $M_1$ is made higher. By use of the formed angle θ calculated in this manner, the direction calculation part 35 can calculate the direction of the capsule medical device 3 (direction of the magnetization direction of the permanent magnet 21) around the f-axis.

As described above, by calculating the direction of the capsule medical device 3 around the f-axis, it is possible to carry out more accurate rotation correction when correcting the image data that rotates along with the rotation of the capsule medical device into the static image data in the data processing part 53.

When a gradient magnetic field is generated as the guidance magnetic field $M_2$ and a magnetic attractive force is applied to the capsule medical device after the rotating magnetic field of the guidance magnetic field $M_2$ is generated, an efficient magnetic attractive force can be generated. That is, by determining the magnetic field direction of the gradient magnetic field according to the direction of the capsule medical device 3 around the f-axis (so as to make the magnetic field direction parallel to the magnetization direction of the permanent magnet 21), it is possible to apply the magnetic attractive force efficiently to the permanent magnet and to generate the magnetic attractive force efficiently.

Since the capsule medical device 3 in the subject's body is located in the direction detection magnetic field $M_1$ formed by the magnetic field generation part 45, the permanent magnet 21 in the capsule medical device 3 is rotated around the f-axis by the direction detection magnetic field $M_1$. The rotation angle α of this permanent magnet 21 (capsule medical device 3) is detected from the images captured by the imaging part 9. The rotation phase of the capsule medical device 3 around the f-axis is calculated from the magnetic field direction of the direction detection magnetic field $M_1$ and the detected rotation angle α of the permanent magnet 21 in the capsule medical device 3, and thereby can be obtained accurately compared with the method of obtaining the rotation amount of the capsule medical device 3 by accumulation.

In other words, by applying the direction detection magnetic field $M_1$ to the permanent magnet 21, the capsule medical device 3 is rotated around the f-axis and the rotation angle α of the capsule medical device 3 is detected from the images captured by the imaging part 9. The directions of the u-axis and the r-axis are calculated from the detected rotation angle α.

Since the axis directions of the r-axis and the f-axis, among the axis directions of the u-axis, the r-axis, and the f-axis in the capsule medical device 3, are calculated from the rotation angle α of the permanent magnet 21 due to the direction detection magnetic field $M_1$, it is not necessary to provide another detection part separately in the capsule medical device 3, and it is thus possible to simplify the configuration thereof.

Since the rotation angle α of the capsule medical device 3 due to the direction detection magnetic field $M_1$ is detected by comparing at least two images acquired by the imaging part 9, the capsule medical device 3 does not need another separate detection part or the like, and the configuration thereof is simplified, resulting in miniaturization of the capsule medical device 3.

Since a plurality of sets of information regarding the rotation of the permanent magnet 21, which is necessary for the direction detection of the capsule medical device 3, is acquired by increasing the magnetic field intensity of the direction detection magnetic field $M_1$ from $H_1$ to $H_2$, the configuration of the medical device guidance system 1 can be simplified while maintaining a high position detection accuracy of the capsule medical device 3.

The guidance magnetic field $M_2$ can be applied to the permanent magnet 21 efficiently, since the magnetic field direction of the guidance magnetic field $M_2$ generated from the magnetic field generation part 45 is adjusted to be parallel to the magnetization direction of the permanent magnet 21, according to the rotation phase of the capsule medical device 3 around the f-axis obtained by the direction calculation part 35. The guidance magnetic field $M_2$ can be applied in the magnetization direction of the permanent magnet 21 as intended, and the capsule medical device 3 can be controlled stably.

Note that the above control of the guidance magnetic field $M_2$ may be carried out also by a control method described in the following; it is not particularly limited.

When the capsule medical device 3 is rotated around the f-axis to be guided by the generation of the guidance magnetic field $M_2$, which is a rotating magnetic field, electric current supplied to the magnetic field generation part 45 changes during the generation of the guidance magnetic field $M_2$. When the supplied current changes in this manner, the guidance magnetic field $M_2$ having a high magnetic field intensity is not generated in order to avoid an excessively large load on the power supply 39.

When the generation of the guidance magnetic field $M_2$ is interrupted in such a situation, the capsule medical device 3 stops moving while the angle formed by the magnetization direction of the permanent magnet 21 and the magnetic field direction of the guidance magnetic field $M_2$ maintains a large value. Then, when the guidance magnetic field $M_2$ of the gradient magnetic field is generated subsequently, the magnetic attractive force applied to the permanent magnet 21 becomes weak.

Accordingly, by generating a static, strong magnetic field immediately before the guidance magnetic field $M_2$ of the rotating magnetic field is interrupted, it is possible to reduce the angle (shift amount) formed by the magnetization direction of the permanent magnet 21 and the magnetic field direction of the guidance magnetic field $M_2$ when the

generation of the guidance magnetic field $M_2$ is interrupted. Then, when controlling the position of the capsule medical device 3 subsequently with the magnetic attractive force, it is possible to generate the attractive force efficiently for the permanent magnet 21 and to control the position of the capsule medical device 3 accurately.

[First modification of the first embodiment]

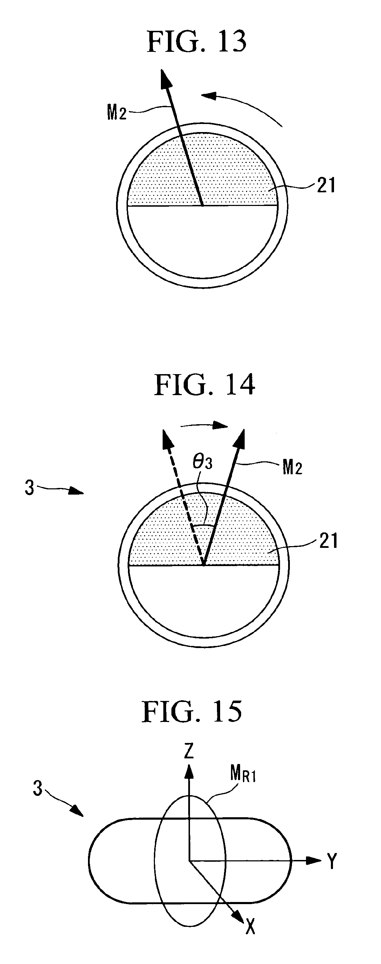

Next, a first modification of the first embodiment of the present invention will be described with reference to Fig. 13 and Fig. 14.
While the basic configuration of a medical device guidance system in the present modification is the same as that in the first embodiment, a method of calculating the magnetization direction of the permanent magnet is different from that in the first embodiment. Accordingly, in the present modification, only the method of calculating the magnetization direction of the permanent magnet will be described by use of Fig. 13 and Fig. 14, and a description of the medical device guidance system configuration etc. will be omitted.
Fig. 13 and Fig. 14 are diagrams illustrating a method of detecting the angle shift between the magnetic field direction of the direction detection magnetic field and the magnetization direction of the permanent magnet in the present modification.
Note that the same constituent parts as those of the first embodiment are denoted by the same symbols, and a description thereof will be omitted. The outlines of a method of guiding the capsule medical device 3 and a method of acquiring an image are also the same as those in the first embodiment, and a description thereof will be omitted.
Here, the detection method of rotation phase of the capsule medical device 3 around the f-axis, that is, the angle shift between the magnetic field direction of the guidance magnetic field $M_2$ and the magnetization direction of the permanent magnet will be described as a feature of the present modification.
For the guidance of the capsule medical device 3, as in the first embodiment, the capsule medical device 3 is rotationally driven and guided to a desired position by the guidance magnetic field $M_2$, which is a rotating magnetic field. In such a case, when the guidance of the capsule medical device 3 is interrupted, that is, the rotation of the guidance magnetic field $M_2$ is interrupted, the magnetic field direction of the guidance magnetic field $M_2$ and the magnetization direction of the permanent magnet 21 have a relationship shown in Fig. 13.
The guidance magnetic field $M_2$ is a magnetic field that rotates around the f-axis of the capsule medical device 3.
Fig. 13 shows a case in which the capsule medical device 3 is rotationally driven by the guidance magnetic field $M_2$ rotating counterclockwise, which is a state where the magnetic field direction of the guidance magnetic field $M_2$ is advanced counter-clockwise against the magnetization direction of the permanent magnet 21. By such advancement of the magnetic field direction of the guidance magnetic field $M_2$ against the magnetization direction of the permanent magnet 21, a rotational torque rotating the permanent magnet 21 is generated in the permanent magnet 21.
After that, the guidance magnetic field $M_2$ is rotated clockwise without changing the magnetic field intensity, as shown in Fig. 14. At this time, the measurement of the rotation angle of the guidance magnetic field $M_2$ is started from a phase where the rotation thereof was stopped.
Then, the rotation angle $\theta_3$ of the guidance magnetic field $M_2$ is measured when the capsule medical device 3 starts to rotate clockwise during observation of the image captured by the imaging part 9 of the capsule medical device 3.
Here, the rotational torque required for rotating the capsule medical device 3 counterclockwise and the rotational torque to rotate it clockwise are assumed to be substantially the same. Therefore, an angle half the rotation angle $\theta_3$ becomes the angle (angle shift) formed by the magnetic field direction of the guidance magnetic field M2 and the magnetization direction of the permanent magnet 21.
Accordingly, the direction calculation part 35 calculates a direction rotated clockwise by $\theta_3/2$ from the magnetic field direction where rotation of the guidance magnetic field $M_2$ was interrupted, as the magnetization direction of the permanent magnet 21, that is, the rotation phase of the capsule medical device 3 around the f-axis.
With the above configuration, the angle formed by the magnetization direction of the permanent magnet 21 and the magnetic field direction of the guidance magnetic field $M_2$ can be calculated simply by reversing the rotation direction in the guidance magnetic field $M_2$ of the rotating magnetic field. Therefore, the control method for generating the magnetic field becomes simple compared with the first embodiment.
The image processing may detect only the start of rotation of the capsule medical device 3. Accordingly, pattern matching of the image does not become complicated as in the first embodiment, and it becomes possible to carry out the angle detection accurately in a shorter time.
Note that the rotating magnetic field may be applied by the guidance magnetic field $M_2$, as described above, or by the direction detection magnetic field $M_1$; it is not particularly limited.

[Second modification of the first embodiment]

Next, a second modification of the first embodiment of the present invention will be described with reference to Fig. 15 to Fig. 20.

While the basic configuration of a medical device guidance system in the present modification is the same as that in the first embodiment, a method of calculating the magnetization direction of the permanent magnet is different from that in the first embodiment. Accordingly, in the present modification, only the method of calculating the magnetization direction of the permanent magnet will be described by use of Fig. 15 to Fig. 20, and a description of the medical device guidance system configuration etc. will be omitted.

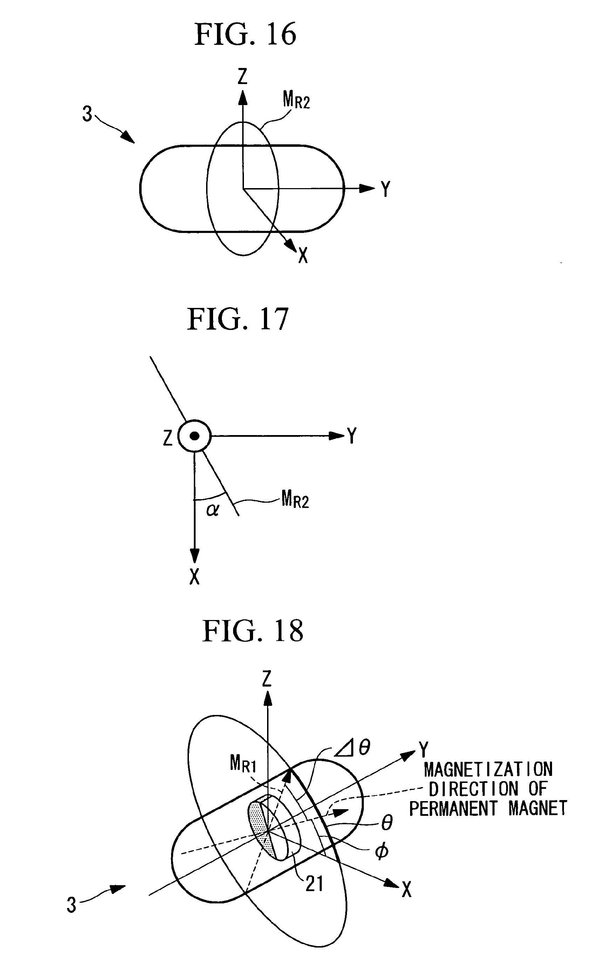

Fig. 15 is a perspective view illustrating a rotating magnetic field $M_{R1}$ formed when the magnetization direction of the permanent magnet is to be obtained in the present modification. Fig. 16 is a perspective view illustrating a rotating magnetic field $M_{R2}$ formed when the magnetization direction of the permanent magnet is to be obtained in the present modification. Fig. 17 is a top view of the rotating magnetic field of Fig. 16, when viewed along the Z-axis.

Note that the same constituent parts as those of the first embodiment are denoted by the same symbols, and a description thereof will be omitted. The outlines of a method of guiding the capsule medical device 3 and a method of acquiring an image are also the same as those in the first embodiment, and a description thereof will be omitted.

Here, the method of detecting the rotation phase of the capsule medical device 3 around a Y-axis, that is, the angle shift between the magnetic field direction of the rotating magnetic field $M_R$ and the magnetization direction of the permanent magnet, will be described as a feature of the present modification.

First, as shown in Fig. 15, the rotating magnetic field $M_{R1}$ is generated to rotate around the Y-axis in a Z-X plane of the capsule medical device 3. Next, as shown in Fig. 16 and Fig. 17, the rotating magnetic field $M_{R2}$ is generated to rotate in a plane that is rotated by an angle $\alpha$ from the Z-X plane of the capsule medical device 3 (hereinafter denoted by "rotation plane"), with an intersection of the Y-axis and the rotation plane as the center.

Here, the X-axis, the Y-axis, and the Z axis in Fig. 16 and Fig. 17 correspond to the r-axis, the f-axis, and the u-axis, respectively.

The capsule is rotated by each of the rotating magnetic fields $M_{R1}$ and $M_{R2}$, and each of the images is acquired when angles between the directions of the generated magnetic fields and the X-Y plane are $\theta$ (when the directions of the rotating fields are the same).

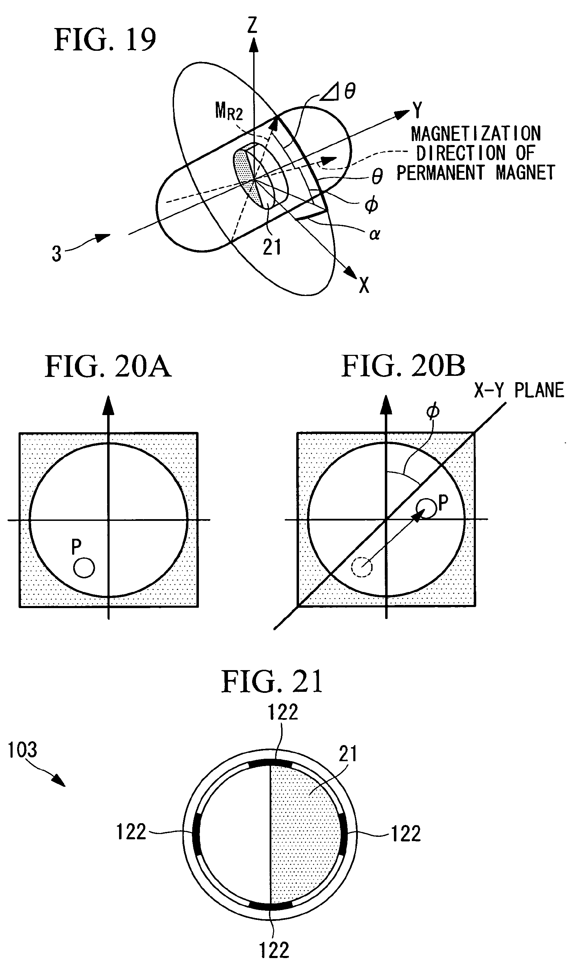

Fig. 18 and Fig. 19 are diagrams respectively showing relationships between the generated magnetic fields and the capsule medical device 3 or the permanent magnet 21 when the rotating magnetic fields $M_{R1}$ and $M_{R2}$ are applied and the images are acquired.

Fig. 20 compares the images captured by the image sensor 27 of the capsule medical device 3 when the directions of the magnetic fields generated by the rotating fields $M_{R1}$ and $M_{R2}$ are the same, and Fig. 20(a) and Fig. 20(b) show the images for the rotating magnetic field $M_{R1}$ and the rotating magnetic field $M_{R2}$, respectively.

By pattern matching of the two acquired images, a moving direction of the image (direction in which the capsule medical device 3 is moved by the change of the rotating magnetic field direction) can be detected. Further, an angle difference $\varphi$ between the detected moving direction and the magnetization direction of the permanent magnet 21, the position of which is determined relative to the image sensor 27 acquiring the images, is obtained. Here, since the detected moving direction is parallel to the X-Y plane, the obtained angle difference $\varphi$ is an angle difference between the magnetization direction of the permanent magnet 21 and the X-Y plane.

As shown in Fig. 18 and Fig. 19, from the angle difference $\theta$ between the direction of the magnetic field generated by the rotating magnetic field $M_{R1}$ or $M_{R2}$ and the X-Y plane and the angle difference $\varphi$ between the permanent magnet and the X-Y plane, an angle difference $\Delta\theta$ between the direction of the generated magnetic field and the magnetization direction of the permanent magnet 21 is obtained by the following formula (4).

$$\Delta\theta = \theta - \varphi \qquad \cdots \quad (4)$$

From the direction of the generated magnetic field and the angle difference $\Delta\theta$ between the direction of the generated magnetic field and the magnetization direction of the permanent magnet 21, the rotation phase of the capsule medical device 3 around the Y-axis can be obtained.

Note that, when the rotating magnetic fields $M_{R1}$ and $M_{R2}$ are generated and the capsule medical device 3 acquires the images, the angle differences between the directions of the generated magnetic fields and the X-Y plane may be different from each other. In this case, by relatively rotating the image captured when the rotating magnetic field $M_{R1}$ is generated and the image captured when the rotating magnetic field $M_{R2}$ is generated, a difference in the angle differences between the directions of the generated magnetic fields and the X-Y plane, which is found when the images are acquired, is cancelled, and processing can be carried out as if the images acquired when the angle differences between the directions of the generated magnetic fields and the X-Y plane are the same.

With the above configuration, when the rotating magnetic fields $M_{R1}$ and $M_{R2}$ are generated, and the angle difference between the directions of the rotating fields and the X-Y plane is $\theta$, the angle difference $\varphi$ between the magnetic

field direction of the permanent magnet 21 and the X-Y plane is obtained from the respective images captured by the image sensor 27 of the capsule medical device 3, and the angle difference $\Delta\theta$ between the direction of the generated magnetic field and the magnetization direction of the permanent magnet 21 can be obtained.

The process to calculate $\Delta\theta$ does not include the angle $\alpha$ formed by the rotating magnetic fields $M_{R1}$ and $M_{R2}$. Thereby, when the formed angle $\Delta\theta$ is obtained, the angle (formed angle $\alpha$) between the planes of the rotating magnetic fields $M_{R1}$ and $M_{R2}$ (rotation planes) does not have a restriction, and the rotating magnetic fields $M_{R1}$ and $M_{R2}$ become easy to control.

Note that the above described detection of the magnetization direction of the permanent magnet 21 may be separated from the guidance of the capsule medical device 3 and carried out independently, or the above described detection of the magnetization direction of the permanent magnet 21 may be carried out when the Y-axis direction of the capsule medical device 3 is changed during guidance of the capsule medical device 3.

That is, when the Y-axis direction of the capsule medical device 3 is changed, an angle difference is generated between a plane perpendicular to the Y-axis (Z-X plane) of the capsule medical device 3 and a plane of the guidance magnetic field $M_{R2}$ of the rotating magnetic field. Using this angle difference, it is possible to carry out the above described detection of the magnetization direction of the permanent magnet 21. Since the above described detection of the magnetization direction of the permanent magnet 21 can be carried out during the guidance of the capsule medical device 3, it is possible to improve efficiency in the guidance and the position detection of the capsule medical device 3.

Note that the rotating magnetic fields $M_{R1}$ and $M_{R2}$ may be the guidance magnetic fields $M_2$ or the direction detection magnetic fields $M_1$; they are not particularly limited.

[Third modification of the first embodiment]

Next, a third modification of the first embodiment of the present invention will be described with reference to Fig. 21 to Fig. 23.

While the basic configuration of a medical device guidance system in the present modification is the same as that in the first embodiment, a method of calculating the magnetization direction of the permanent magnet is different from that in the first embodiment. Accordingly, in the present modification, only the method of calculating the magnetization direction of the permanent magnet will be described by use of Fig. 21 to Fig. 23, and a description of the medical device guidance system configuration etc. will be omitted.

Fig. 21 is a cross-sectional view illustrating the configuration of a permanent magnet and a surrounding part thereof in a capsule medical device of the present modification.

Note that the same constituent parts as those in the first embodiment are denoted by the same symbols, and a description thereof will be omitted.

The capsule medical device 103 is provided with a pressure sensor (magnetic force measurement part) 122 around a permanent magnet 21, as shown in Fig. 21. The pressure sensor 122 detects the magnetic attractive force applied to the permanent magnet 21. The present modification will be described as applied to an example in which four of the pressure sensors 122 are disposed at equal intervals around the permanent magnet 21.

Here, the method of detecting the rotation phase of the capsule medical device 103 around the f-axis, that is, the angle shift between the magnetic field direction of the gradient magnetic field $M_s$ and the magnetization direction of the permanent magnet 21, will be described as a feature of the present modification. Note that the outlines of a method of guiding the capsule medical device 103 and a method of acquiring the image are the same as those in the first embodiment, and a description thereof will be omitted.

For the guidance of the capsule medical device 103, the capsule medical device 103 is rotationally driven by the guidance magnetic field $M_2$, which is a rotating magnetic field, and is guided to a desired position, as in the first embodiment. After that, the generation of the guidance magnetic field $M_2$ is interrupted.

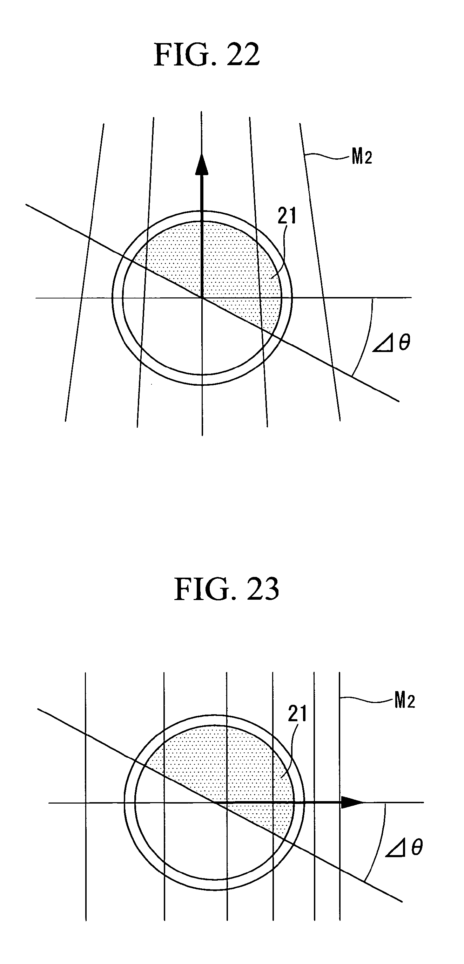

Fig. 22 is a diagram illustrating a state in which a gradient magnetic field is applied to the capsule medical device of Fig. 21. Fig. 23 is a diagram illustrating a state in which another gradient magnetic field is applied to the capsule medical device of Fig. 21.

The gradient magnetic field Ms is generated as shown in Fig. 22 after the guidance magnetic field $M_2$ is interrupted, and the magnetic attractive force F is applied to the permanent magnet 21 in the magnetic field direction of the guidance magnetic field $M_2$ at the time of interruption. Here, the gradient magnetic field shown in Fig. 22 is a gradient magnetic field that has magnetic force lines extending along the direction of the guidance magnetic field M2 at the time of interruption and also a higher intensity of the magnetic force lines in the above magnetic field direction. An angle $\Delta\theta$ is formed by the magnetization direction of the permanent magnet 21 and the magnetic field direction of the guidance magnetic field $M_2$ at the time of interruption.

Alternatively, the gradient magnetic field $M_s$ is generated in a manner as shown in Fig. 23, and the magnetic force F is applied to the permanent magnet 21 in the magnetic field direction of the guidance magnetic field $M_2$ at the time of interruption. Here, the gradient magnetic field shown in Fig. 23 is a gradient magnetic field that has magnetic force lines extending along the magnetic field direction of the guidance magnetic field $M_2$ at the time of interruption and also a higher intensity of the magnetic force lines in a direction perpendicular to the above magnetic field direction.

The magnetic attractive force F applied to the permanent magnet 21 is detected by the pressure sensor 122. The detected magnetic attractive force F is compared with a theoretical magnetic attractive force $F_0$ calculated when the magnetization direction of the permanent magnet 21 and the force direction of the magnetic attractive force are the same. Specifically, the magnetic attractive force F is compared with the theoretical magnetic attractive force $F_0$, and $\Delta\theta$ is obtained from the following formula (5).

$$F = F_0 \cdot \cos \Delta\theta \quad \cdots \quad (5)$$

From $\Delta\theta$ obtained in this manner and the magnetic field direction of the guidance magnetic field $M_2$ at the time of interruption, the magnetization direction of the permanent magnet 21 is calculated.

With the above configuration, the part generating the gradient magnetic field for guiding the capsule medical device 103 in the magnetic field generation part 45 can be used for generating the gradient magnetic field $M_s$ used for detecting the magnetization direction of the permanent magnet 21, and thereby the configuration of the magnetic field generation part 45 can be simplified.

In other words, from the magnetic attractive force F (response) that is applied to the permanent magnet 21 by the gradient magnetic field $M_s$ formed for the direction detection, the phase of the capsule medical device 103 around the f-axis is calculated. Accordingly, the magnetic field generation part 45 can be shared as the generation parts forming the gradient magnetic field $M_s$ and another uniform magnetic field guiding the capsule medical device 103.

The magnetic attractive force applied to the permanent magnet 21 is directly detected by the pressure sensor 122 as a pressure, and this improves the calculation accuracy in the phase of the capsule medical device 103 around the f-axis.

The position detection calculation and the image processing become unnecessary, and thereby it becomes easy to perform the data processing for obtaining the position etc. of the capsule medical device 103.

Since the magnetic attractive force applied to the permanent magnet 21 is transferred to the capsule medical device 103 via the pressure sensor 122, the guidance of the capsule medical device 103 can be continued.

[Fourth modification of the first embodiment]

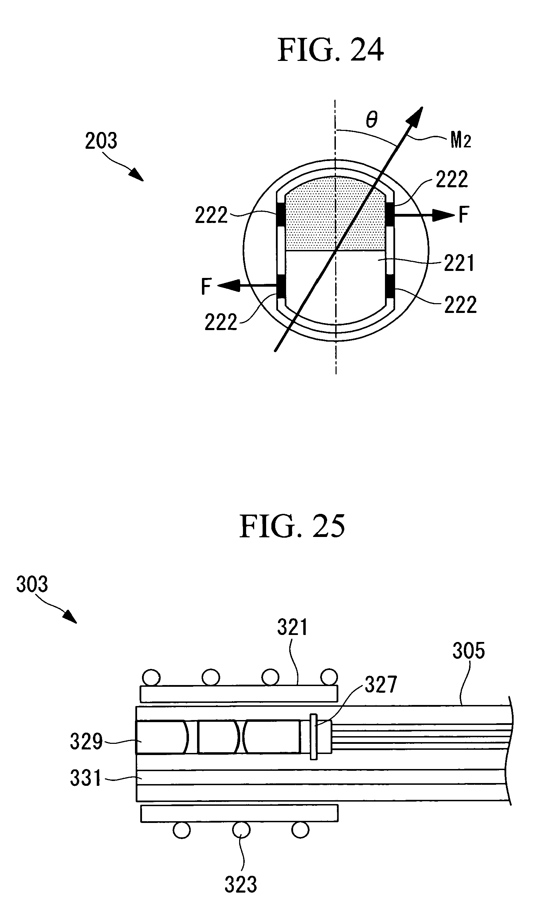

Next, a fourth modification of the first embodiment of the present invention will be described with reference to Fig. 24.

While the basic configuration of a medical device guidance system in the present modification is the same as that in the first embodiment, a method of calculating the magnetization direction of the permanent magnet is different from that in the first embodiment. Accordingly, in the present modification, only the method of calculating the magnetization direction of the permanent magnet will be described by use of Fig. 24, and a description of the medical device guidance system configuration etc. will be omitted.

Fig. 24 is a cross-sectional view illustrating the configuration of a permanent magnet and a surrounding part thereof in a capsule medical device of the present modification.

Note that the same constituent parts as those in the first embodiment are denoted by the same symbols, and a description thereof will be omitted.

A capsule medical device 203 is provided with a force sensor (magnetic force measurement part) 222 disposed around a permanent magnet 221, as shown in Fig. 24. The present modification will be described as applied to a permanent magnet that has a pair of faces formed parallel to the magnetization direction. The force sensor 222 detects the magnetic attractive force applied to the permanent magnet 221. The present modification will be described as applied to an example in which a total of four of the force sensors 222 are disposed on the pair of faces.

Here, the method of detecting the rotation phase of the capsule medical device 203 around the f-axis, that is, the angle shift between the magnetic field direction of the guidance magnetic field $M_2$ and the magnetization direction of the permanent magnet 221, will be described as a feature of the present modification. Note that the outlines of a method of guiding the capsule medical device 203 and a method of acquiring the image are the same as those in the first embodiment, and a description thereof will be omitted.

For the guidance of the capsule medical device 203, the capsule medical device 203 is rotationally driven by the guidance magnetic field $M_2$, which is a rotating field, and is guided to a desired position, as in the first embodiment.

At this time, a rotational torque T is generated in the permanent magnet 221 by the guidance magnetic field $M_2$, and the capsule medical device 203 is rotationally driven by this rotational torque T.

The force sensor 222 detects the force F by the pressure from the rotationally driven permanent magnet 221. A detection signal of the force sensor 222 is superimposed on the image data captured by the imaging part 9 and is transmitted to the image data receiving part 43 in the external device 5.

The force F detected by the force sensor 222 is used for the calculation of the rotational torque T generated in the permanent magnet 221 according to arrangement positions of the force sensors 222. Then, the angle $\theta$ formed by the magnetization direction of the permanent magnet 221 and the magnetic field direction of the guidance magnetic field $M_2$ is calculated from a relational expression (formula (6)) among the calculated rotational torque T, a magnetic field vector M of the permanent magnet 221 and a magnetic field vector H of the guidance magnetic field $M_2$.

$$T = MH \cdot \cos \theta \qquad \cdots \quad (6)$$

The magnetization direction of the permanent magnet 221 is calculated from $\theta$ obtained in this manner and the magnetic field direction of the guidance magnetic field $M_2$.

With the above configuration, the angle formed by the magnetization direction of the permanent magnet 221 and the magnetic field direction of the guidance magnetic field $M_2$ is calculated during the guidance of the capsule medical device 203, and the magnetization direction of the permanent magnet 221 can be calculated.

Since the rotational torque T generated in the permanent magnet 221 is directly measured by the force sensor 222, it is possible to accurately calculate the magnetization direction of the permanent magnet 221.

The data processing becomes easy to carry out in the calculation of the magnetization direction of the permanent magnet 221, because the position detection calculation and the image processing are not necessary. Accordingly, responsiveness of the calculation for the magnetization direction of the permanent magnet 221 becomes better, and controllability in the generation direction of the gradient magnetic field and controllability in the rotation correction of the acquired image data are improved.

Fig. 25 is a diagram illustrating a case where, instead of the capsule medical device, an endoscope device is used for the medical device applied to the medical device guidance system of the present invention.

Note that, while the above described first embodiment to the fourth modification of the first embodiment are described for a case where a capsule medical device is used as the medical device, an endoscope device 303 may be used, as shown in Fig. 25; the medical device is not particularly limited.

The endoscope device (medical device) 303 is provided with an endoscope 305 that is inserted into a body cavity of a subject, a permanent magnet (magnetic field response part) 321 guiding a front end of the endoscope 305, and a spiral part 323 generating a forward or backward drive force, as shown in Fig. 25.

The endoscope 305 is provided with an image sensor 327 imaging the inside of the body cavity, a lens group 329 forming an image of the body cavity onto the image sensor 327, and a forceps hole 331 guiding forceps to the front end of the endoscope 305.

The permanent magnet 321 is formed in a cylindrical shape so as to have the endoscope 305 inserted therein and is magnetized in a radial direction (e.g., vertical direction in Fig. 25). The permanent magnet 321 is disposed rotatably with respect to the endoscope 305 around the center axis thereof, and also movement thereof in the center axis direction is restricted.

The spiral part 323 is disposed spirally on the outer peripheral surface of the permanent magnet 321, and is disposed together with the permanent magnet 321 rotatably with respect to the endoscope 305.

The medical device guidance system of the present invention using such an endoscope device 303 can detect the position and direction of the endoscope device 303 accurately, as in the case where the capsule medical device is used, and can carry out guidance control of the endoscope device 303 stably and efficiently.

[Second embodiment]

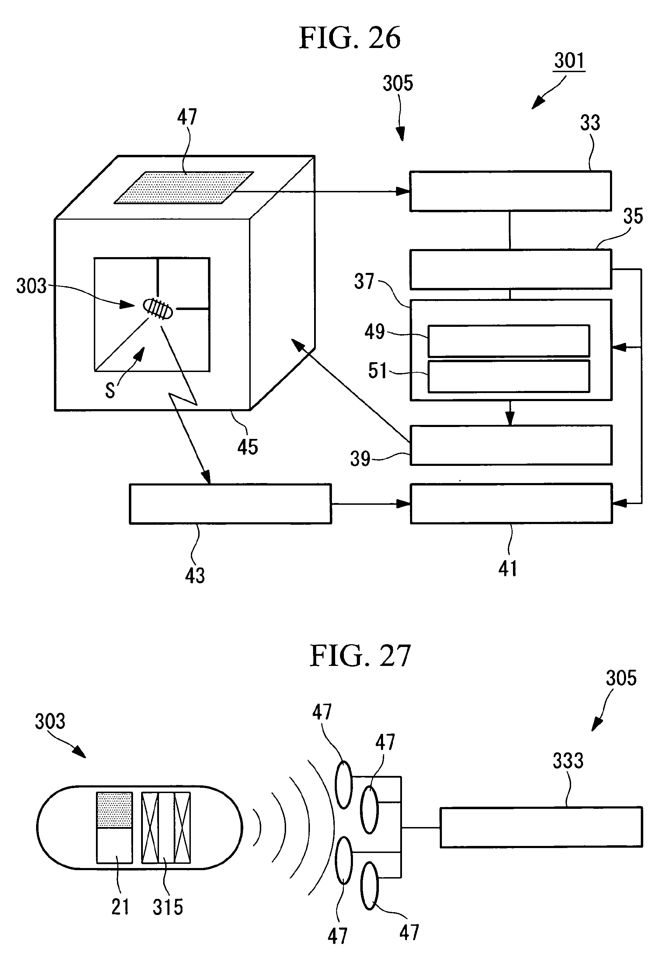

Next, a second embodiment of the present invention will be described with reference to Fig. 26 to Fig. 29.

While the basic configuration of a medical device guidance system in the present embodiment is the same as that in the first embodiment, a method of detecting the f-axis direction of the capsule medical device is different from that in the first embodiment. Accordingly, in the present embodiment, only the method of detecting the f-axis direction of the capsule medical device will be described by use of Fig. 26 to Fig. 29, and a description of the medical device guidance system configuration etc. will be omitted.

Fig. 26 shows a schematic diagram illustrating, in outline, a medical device guidance system according to the present embodiment.

Note that the same constituent parts as those in the first embodiment are denoted by the same symbols, and a description thereof will be omitted.

A medical device guidance system 301 is provided with a capsule medical device (medical device) 303 introduced into a body cavity of a subject and an external device 305 detecting the position and direction of the capsule medical device 303 and also guiding the capsule medical device 303, as shown in Fig. 26.

Fig. 27 shows a schematic diagram illustrating parts different from those of the first embodiment in the medical device guidance system of Fig. 26.

The capsule medical device 303 is provided with a permanent magnet 21, the magnetization direction of which corresponds to a radial direction of the capsule medical device 303, and an oscillation coil 315, the center axis line of which corresponds to the magnetization direction of the permanent magnet 21, as shown in Fig. 27.

A position and orientation detection part 333 of the external device 305 detects five degrees freedom of coordinate values (position) of the capsule medical device 303 in a coordinate system of the external device 305 and a direction of the u-axis in the capsule medical device 303, that is, the rotation phases of the capsule medical device 303 around the f-axis and the r-axis. A method of calculating these coordinate values and the rotation phase can be realized by a publicly known calculation method and is not particularly limited. The position and orientation detection part 333 is provided with a plurality of detection coils 47 and receives detection signals from the detection coils 47.

A direction calculation part 335 calculates the rotation phase of the capsule medical device 303 around the u-axis (direction of the medical device), that is, the direction of the f-axis and the r-axis in the capsule medical device 303, as shown in Fig. 26. The calculation method will be described below.

Next, a method of detecting the f-axis direction in the capsule medical device 303 will be described as a feature of the present embodiment. Note that the outlines of a method of guiding the capsule medical device 303 and a method of acquiring the image are the same as those in the first embodiment, and a description thereof will be omitted.

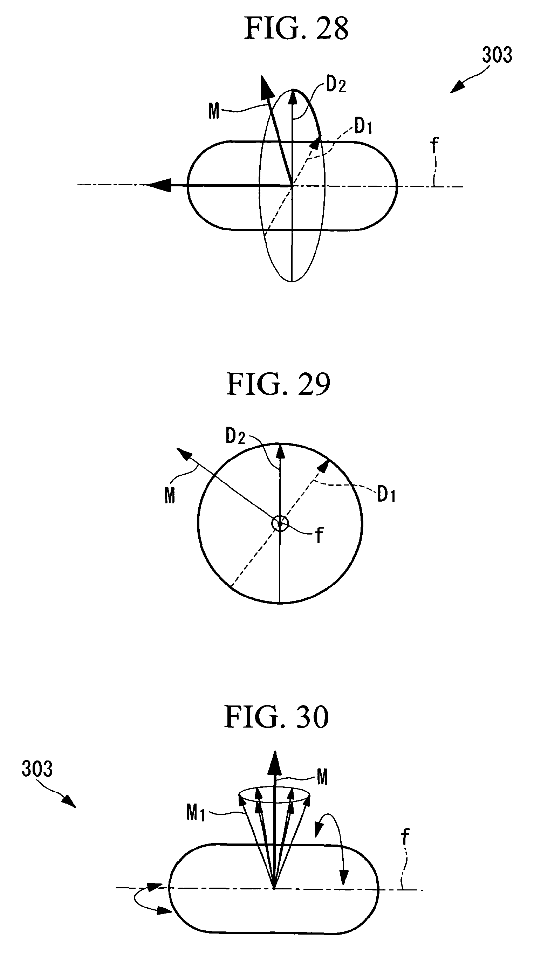

Fig. 28 is a schematic diagram illustrating the relationships among the magnetic field direction of a static magnetic field applied to the capsule medical device of Fig. 26, the magnetization direction of the permanent magnet, and the f-axis direction of the capsule medical device. Fig. 29 is a diagram illustrating the relationship between the magnetic field direction of the static magnetic field applied to the capsule medical device and the magnetization direction of the permanent magnet, viewed in the f-axis direction of Fig. 28.

First, a static magnetic field M having the magnetic field direction close to the magnetization direction $D_1$ of the permanent magnet 21 is generated, as shown in Fig. 28 and Fig. 29. At this time, the magnetic field intensity of the static magnetic field M is preferably higher than a magnetic field intensity generating a torque required to rotate the capsule medical device 303 around the f-axis, and lower than a magnetic field intensity required to move (turn) the f-axis direction of the capsule medical device 303.

A torque is applied to the permanent magnet 21 by the static magnetic field M generated in this manner, and the capsule medical device 303 rotates. Here, because of the shape and non-uniformity of the capsule medical device 303, the rotation amount of the capsule medical device 303 around the f-axis is considerably larger than the rotation amount around the u-axis or the r-axis. Accordingly, the capsule medical device 303 is assumed to rotate around the f-axis by the static magnetic field M.

Specifically, the permanent magnet 21 (capsule medical device 303) rotates from the direction $D_1$ before the generation of the static magnetic field M to a direction $D_2$ by the generation of the static magnetic field M.

This rotation of the permanent magnet 21 is detected by the position and orientation detection part 333, and a rotation plane (u-r plane) of the capsule medical device 303 is calculated by use of the detected rotation from the direction $D_1$ to the direction $D_2$. The f-axis has a perpendicular positional relationship with respect to the calculated rotation plane, and thereby the f-axis direction can be obtained from the calculated rotation plane.

When guiding the capsule medical device 303 by the magnetic attractive force, it is possible to determine the direction of the magnetic gradient to efficiently generate the magnetic attractive force according to the f-axis direction obtained as described above.

When rotating the capsule medical device 303 around the f-axis, it is possible to generate the rotating magnetic field in the u-r plane obtained as described above.

Note that the magnetic field intensity of the static magnetic field M to be generated may be lower than that of the guidance magnetic field $M_2$.

Thereby, it is possible to detect the f-axis direction correctly while causing little turning of the capsule medical device 303.

As described above, the static magnetic field M may be generated for detecting the f-axis direction of the capsule medical device 303, or the f-axis direction may be detected continuously by use of the guidance magnetic field M2 generated for guiding the capsule medical device 303; the method is not particularly limited.

When the f-axis direction is detected in this manner while the rotating magnetic field is being generated, the f-axis direction of the capsule medical device 303 can be detected efficiently during guidance.

With the above configuration, it becomes possible to carry out the position and orientation detection of the capsule medical device 303 having six degrees of freedom by using the medical device guidance system 301 having the position and orientation detection part 333 which can detect five degrees of freedom. That is, it is possible to obtain the f-axis direction of the capsule medical device 303.

Since the f-axis direction of the capsule medical device 303 is obtained by the generation of the static magnetic field in one direction, this method is efficient compared with the other methods.

Since the f-axis direction of the capsule medical device 303 can be obtained by the single generation of the detection magnetic field, the obtained information of the f-axis direction is efficiently fed back to the guidance control of the capsule medical device 303.

[First modification of the second embodiment]

Next, a first modification of the second embodiment in the present invention will be described with reference to Fig. 30 to Fig. 32.

While the basic configuration of a medical device guidance system in the present modification is the same as that in the second embodiment, a method of detecting the f-axis direction in the capsule medical device is different from that in the second embodiment. Accordingly, in the present modification, only the method of detecting the f-axis direction of the capsule medical device will be described by use of Fig. 30 to Fig. 32, and a description of the medical device guidance system configuration etc. will be omitted.

Fig. 30 is a schematic diagram illustrating the magnetic field direction of a magnetic field generated in the direction detection of the present modification.

Note that the same constituent parts as those in the second embodiment are denoted by the same symbols and a description thereof will be omitted. The outlines of a method of guiding the capsule medical device 303 and a method of acquiring the image are the same as those in the second embodiment, and a description thereof will be omitted.

Here, the method of detecting the f-axis direction in the capsule medical device 303 will be described as a feature of the present modification.

First, a plurality of direction detection magnetic fields $M_1$ are generated, having the same angle difference relative to the magnetization direction M of the permanent magnet 21 (center axis line direction of the oscillation coil 315) preliminarily detected by the position and orientation detection part 333.

The direction of the capsule medical device 3 is changed around the f-axis, the u-axis, and the r-axis by the generated direction detection magnetic field $M_1$. At this time, because of the shape and the non-uniformity of the capsule medical device, the rotation of the capsule medical device around the f-axis has a better response than the rotation around the u-axis or the r-axis.

The magnetization directions M of the permanent magnet 21 are obtained by the position and orientation detection part 333 when the plurality of direction detection magnetic fields M1 is applied, and angle differences are obtained relative to the preliminarily detected magnetization direction M of the permanent magnet 21 before the application of the direction detection magnetic field $M_1$. A plane formed by the magnetic field direction of the direction detection magnetic field $M_1$ maximizing this angle difference and the magnetization direction M of the permanent magnet 21 before the application of the preliminarily detected direction detection magnetic field $M_1$ is calculated as the u-r plane. The f-axis direction is obtained from the calculated rotation plane, since the f-axis has a perpendicular positional relationship with respect to the calculated rotation plane.

When the capsule medical device 303 is guided by the magnetic attractive force, the direction of the magnetic gradient is determined so as to generate the magnetic attractive force efficiently according to the f-axis direction obtained as described above.

Specifically, the rotation plane of the capsule medical device 303 is calculated from the magnetization directions M of the permanent magnet 21 detected by the position and orientation detection part 333, before and after the generation of the direction detection magnetic field $M_1$,

When the capsule medical device 333 is rotated around the f-axis, a rotation magnetic field may be generated in the u-r plane obtained as described above.

While the rotation plane of the capsule medical device 303 may be calculated by using the position and orientation detection part 333 as described above, the rotation plane may be obtained by the pattern matching of images, as will be described below.

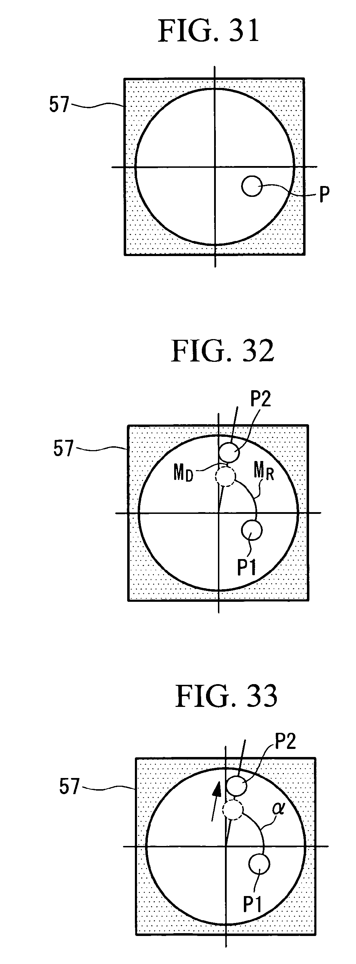

Fig. 31 is a diagram illustrating an image captured before the generation of the plurality of direction detection magnetic fields $M_1$. Fig. 32 is a diagram illustrating an image captured after the generation of the plurality of direction detection magnetic fields $M_1$.

First, before the generation of the plurality of direction detection magnetic fields $M_1$, an inside of a body cavity of a subject is imaged by the imaging part 9, and a particular detection pattern P is set in the captured image, as shown in Fig. 31.

Then, the inside of the body cavity of the subject is imaged by the imaging part 9 while the plurality of direction detection magnetic fields $M_1$ is being generated. In the images captured in this manner, the detection pattern P moves from a position $P_1$ to a position $P_2$, as shown in Fig. 32.

This movement of the detection pattern P is divided into a rotational direction component $M_R$ that moves on a circumference having a rotation center at the center of the image and a radial direction component $M_D$ that moves from the center of the image in a radial direction. A plane formed by the magnetic field direction of the direction detection magnetic field maximizing the rotational direction component $M_R$ of the divided components obtained for each of the plurality of the direction detection magnetic fields $M_1$ and the magnetization direction M of the permanent magnet 21 preliminarily detected by the position and orientation detection part 333 before the generation of the plurality of direction detection magnetic fields $M_1$ is detected as the u-r plane, and the direction perpendicular to the u-r plane is detected as the f axis.

By setting the plurality of detection patterns P, it is possible to obtain the rotational direction component $M_R$ and the radial direction component $M_D$ more accurately.

With the above configuration, it becomes possible to carry out the position and direction detection of the capsule medical device having six degrees of freedom using the medical device guidance system 301 having the position and orientation detection part 333 that detects five degrees of freedom. That is, the f-axis direction of the capsule medical device can be obtained.

Since the f-axis direction of the capsule medical device 303 is calculated by use of the plurality of direction detection magnetic fields $M_1$, the f-axis direction can be obtained accurately compared with the method of using one direction detection magnetic field $M_1$.

[Second modification of the second embodiment]

Next, a second modification of the second embodiment of the present invention will be described with reference to Fig. 33 to Fig. 35.

While the basic configuration of a medical device guidance system in the present modification is the same as that in the second embodiment, a method of detecting the f-axis direction in the capsule medical device is different from that in the second embodiment. Accordingly, in the present modification, only the method of detecting the f-axis direction of the capsule medical device will be described by use of Fig. 33 to Fig. 35, and a description of the medical device guidance system configuration etc. will be omitted.

Fig. 33 is a diagram illustrating the relationships among the magnetization directions of the permanent magnet before and after generation of a magnetic field used for position detection in the present modification and the magnetic field direction of the position detection magnetic field.

Note that the same constituent parts as those in the second embodiment are denoted by the same symbols, and a description thereof will be omitted. The outlines of a method of guiding the capsule medical device 303 and a method of acquiring the image are the same as those in the second embodiment, and a description thereof will be omitted.

Here, the detection method of the f-axis direction in the capsule medical device 303 will be described as a feature of the present modification.

First, a direction detection magnetic field $M_1$ is generated so as to have an angle close to that of the magnetization direction M of the permanent magnet 21 (direction of the center axis line of the oscillation coil 315), which is preliminarily detected by the position and orientation detection part 333. Then, the capsule medical device 303 is rotated by the direction detection magnetic field $M_1$.

Fig. 33 is a diagram illustrating an image captured after the generation of the direction detection magnetic field $M_1$.

Then, as will be described below, a rotation angle $\alpha$ of the capsule medical device 303 in the rotation plane is obtained from the image pattern matching.

Specifically, as shown in Fig. 33, a detection pattern P moves from a position $P_1$ to a position $P_2$ in images captured by the imaging part 9.

This movement of the detection pattern P is divided into a rotational direction component $M_R$ that moves on a circumference having a rotation center at the center of the image and a radial direction component $M_D$ that moves from the center of the image in a radial direction, and the rotation angle $\alpha$ is calculated from the divided rotational direction component $M_R$.

The position and orientation detection part 333 detects the center axis line directions of the oscillation coil 315, that is, the magnetization directions $H_1$ and $H_2$ of the permanent magnet 21, before and after the generation of the direction detection magnetic field $M_1$.

Here, $H_1$ is a vector representing the magnetization direction of the permanent magnet 21 before the generation of the direction detection magnetic field $M_1$ and is expressed as $H_1 = (a1, b1, c1)$. $H_2$ is a vector representing the magnetization direction of the permanent magnet 21 after the generation of the direction detection magnetic field $M_1$ and is expressed as $H2 = (a2, b2, c2)$.

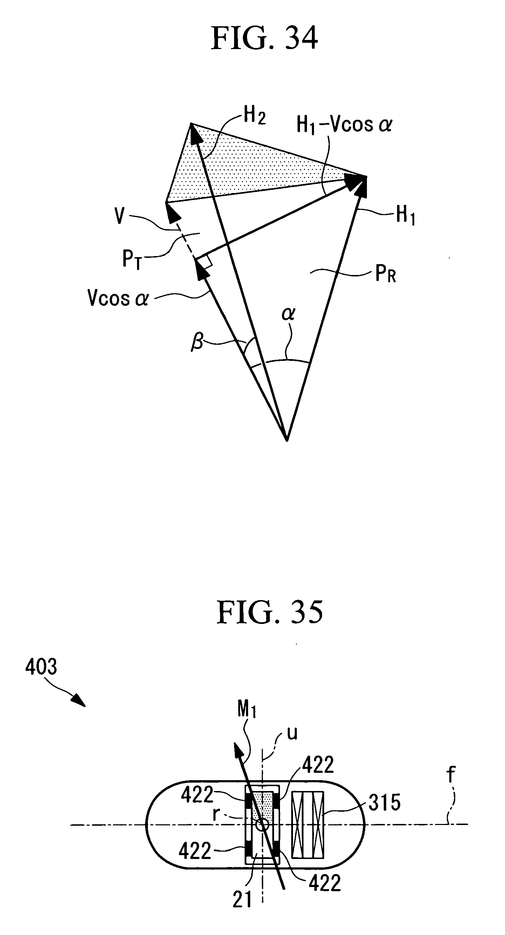

Fig. 34 is a vector diagram illustrating a method of calculating a rotation plane and a turning plane in the capsule medical device of Fig. 33.

When a unit vector along an intersection line of the rotation plane including $H_1$ and the turning plane including $H_2$ is defined as V, the V-$H_1$ plane becomes a rotation plane $P_R$ and the V-$H_2$ plane becomes a turning plane $P_T$, in Fig. 34.

The vector V is a unit vector representing the direction of the vector $H_1$, which is rotated by the rotation angle $\alpha$ along the rotation plane $P_R$, and is expressed as V = (x, y, z).

When the rotation plane $P_R$ is defined as a plane that corresponds to the plane of the paper, the turning plane $P_T$ becomes a plane intersecting the plane of the paper. In other words, the vectors $H_1$ and V are vectors directed along the plane of the paper, and the vector $H_2$ is a vector intersecting the plane of the paper.

Further, an angle formed by the vector $H_1$ and the vector V becomes the above described rotation angle $\alpha$, and an angle formed by the vector $H_2$ and the vector V becomes a turning angle $\beta$.

The following formulas (7), (8), and (9) are derived from the above relationships among the vectors $H_1$, $H_2$, and V. The formula (7) is derived from the result that the angle formed by the vector $H_1$ and the vector V is $\alpha$. The formula (8) is derived from the definition of the vector V as a unit vector. The rotation plane $P_R$ and the turning plane $P_T$ intersect each other perpendicularly. At this time, a vector $(H_1 - V\cdot\cos\alpha)$, which is included in the rotation plane $P_R$ and perpendicular to the intersection line vector $(H_1)$ of the rotation plane $P_R$ and the turning plane $P_T$, becomes perpendicular to any vector $(H_2)$ included in the turning plane $P_T$. Accordingly, the formula (9) is derived from the relationship that the vector $H_2$ and the vector $H - V\cdot\cos\alpha$ are perpendicular to each other.

[Formula 1]

$$H_1 \cdot V = a_1\,x + b_1\,y + c_1\,z = \cos\alpha \qquad \cdots\cdots(7)$$

$$\left|V\right| = x\hat{}2 + y\hat{}2 + z\hat{}2 = 1 \qquad \cdots\cdots(8)$$

$$H_2 \cdot (H_1 - V\cos\alpha) = \begin{bmatrix} a_2, b_2, c_2 \end{bmatrix}\begin{bmatrix} a_2 - x\cos\alpha \\ b_2 - y\cos\alpha \\ c_2 - z\cos\alpha \end{bmatrix} = 0 \qquad \cdots\cdots(9)$$

From the simultaneous equations of the above formulas (7), (8), and (9), two sets of (x, y, z) are obtained. Of the two sets, one set of (x, y, z) is determined uniquely according to the rotation direction and the radial movement direction of the detection pattern P in the images.

From the determined (x, y, z) = V, the rotation plane $P_R$ and the turning plane $P_T$ are calculated, and the f-axis direction of the capsule medical device 303 after the magnetic field generation is calculated.

Note that the calculation of the f-axis direction in the capsule medical device 303 may be carried out continuously while a rotating magnetic field or the like, that is, the guidance magnetic field $M_2$, is continuously being generated.

With the above configuration, it becomes possible to carry out the position and orientation detection of the capsule medical device having six degrees of freedom using the medical device guidance system 301 having the position and orientation detection part 333 that detects five freedoms. That is, the f-axis direction of the capsule medical device can be obtained.

Since the movement amount of the capsule medical device is obtained from the detection information of the position and orientation detection part 333 and the information obtained from the pattern matching of the images captured by the imaging part 9, it is possible to obtain the f-axis direction of the capsule medical device accurately.

Since the f-axis direction of the capsule medical device 303 is obtained by the generation of a static magnetic field in one direction, this method is more efficient than the other methods.

When the f-axis direction is detected while the rotating magnetic field is being generated, the f-axis direction of the capsule medical device 303 can be detected efficiently during guidance.

The f-axis direction of the capsule medical device 303 can be obtained by the single generation of the detection magnetic field, and thereby the obtained information of the f-axis direction is efficiently fed back to the guidance control of the capsule medical device 303.

[Third modification of the second embodiment]

Next, a third modification of the second embodiment of the present invention will be described with reference to Fig. 35 to Fig. 38.

While the basic configuration of a medical device guidance system in the present modification is the same as that in the second embodiment, a method of detecting the f-axis direction in the capsule medical device is different from that in the second embodiment. Accordingly, in the present modification, only the method of detecting the f-axis direction of the capsule medical device will be described by use of Fig. 35 to Fig. 38, and a description of the medical device guidance system configuration etc. will be omitted.

Fig. 35 is a diagram illustrating the configuration of a permanent magnet and a surrounding part thereof in a capsule medical device in the present modification, as well as the magnetic field direction of the direction detection magnetic field applied to the permanent magnet.

Note that the same constituent parts as those in the second embodiment are denoted by the same symbols and a description thereof will be omitted.

A capsule medical device 403 is provided with the permanent magnet 21, the magnetization direction of which corresponds to a radial direction of the capsule medical device 303, and a force sensor (magnetic force measurement part) 422 detecting a rotational torque applied to the permanent magnet 21 around the r-axis (axis perpendicular to the plane of the paper), as shown in Fig. 35.

The force sensor 422 detects the rotational torque applied to the permanent magnet 21. The present modification will be described as applied to an example in which at least four of the force sensors 422 are disposed on a pair of faces perpendicular to the f-axis of the permanent magnet 21.

Here, the method of detecting the f-axis direction in the capsule medical device 403 will be described as a feature of the present modification. Note that the outlines of a method of guiding the capsule medical device 403 and a method of acquiring the image are the same as those in the second modification, and a description thereof will be omitted.

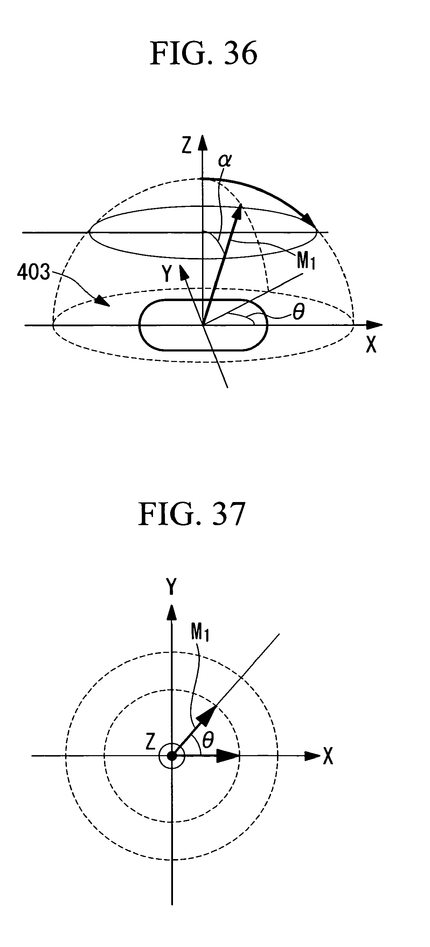

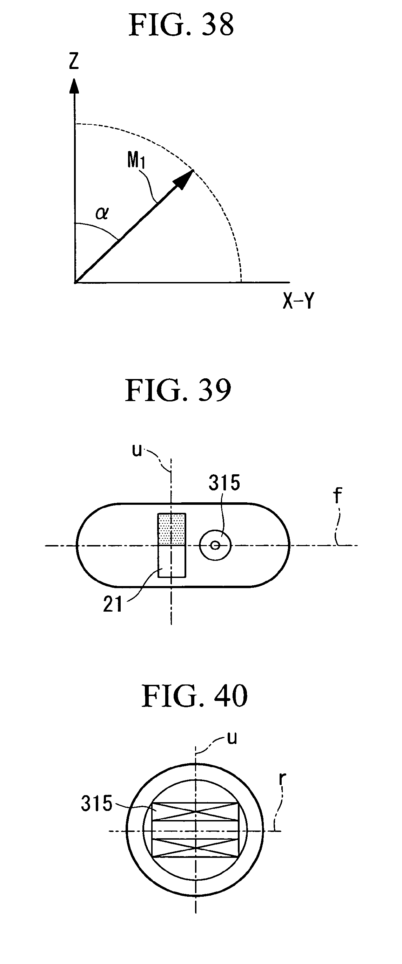

Fig. 36 is a perspective view illustrating the magnetization direction of the direction detection magnetic field of Fig. 35. Fig. 37 is a diagram showing the direction detection magnetic field of Fig. 36, which is viewed in a Z-axis direction. Fig. 38 is a diagram illustrating an angle formed by the magnetization direction of the direction detection magnetic field of Fig. 36 and the Z-axis.

The X-axis, the Y-axis, and the Z axis in Fig. 36 to Fig. 38 correspond to the f-axis, the r-axis, and the u-axis in the capsule medical device, respectively.

First, a direction detection magnetic field $M_1$ is applied having an angle $\alpha$ relative to the magnetization direction of the permanent magnet 21 (Z-axis direction), as shown in Fig. 35 and Fig. 36. A plane including the direction detection magnetic field M1 and the Z-axis is rotated from the X-axis (f-axis of the capsule medical device 403) around the Z-axis by an angle $\theta$, as shown in Fig. 36 and Fig. 37. Further, the direction detection magnetic field $M_1$ is rotated from the Z-axis by an angle $\alpha$ to the X-Y plane side, as shown in Fig. 36 and Fig. 38.

In this manner, when the direction detection magnetic field $M_1$ is applied to the permanent magnet 21, a rotational torque is applied to the permanent magnet 21 and presses the force sensor 422. The pressure force is detected by the force sensor 422, and a detection signal of the force sensor 422 is superimposed on the image data and transmitted to the image data receiving part 43 in the external device.

The rotational torque T applied to the permanent magnet 21 is obtained on the basis of the detection signal of the force sensor 422 and the arrangement of the force sensors 422. Meanwhile, the rotational torque T applied to the permanent magnet 21 is expressed by the following formula (10) using the magnetic field intensity H of the direction detection magnetic field $M_1$, the magnetic field intensity M of the permanent magnet 21, and the above formed angles $\alpha$ and $\theta$.

$$T = HM \cdot \sin \alpha \cdot \cos \theta \quad \cdots (10)$$

By solving this formula (10), two solutions ($\pm x$) are obtained for $\theta$.

Subsequently, a direction detection magnetic field $M_1$ is applied having a magnetization direction different from that of the above direction detection magnetic field $M_1$, and the formed angle $\theta$ is obtained again. By use of the same $\theta$ value of the $\theta$ values obtained two times in the coordinate system of the external device 305, the X-axis direction of the capsule medical device 403 is calculated.

One $\theta$ value may be selected from the two calculated $\theta$ values by use of the preceding guidance history, for example, information such as that indicating on which side the capsule medical device has been turned for guidance, without applying the direction detection magnetic field $M_1$ having the different magnetization direction as described above.

With the above configuration, it becomes possible to carry out the position and orientation detection of the capsule medical device 403 having six degrees of freedom using the medical device guidance system 301 having the position and orientation detection part 333 that detects five degrees of freedom. That is, the X-axis direction of the capsule medical device 403 can be obtained.

Since the rotational torque T generated in the permanent magnet 21 is measured directly by the force sensor 422, the magnetization direction of the permanent magnet 21 can be calculated accurately.

The position detection calculation and the image processing are not necessary, and thereby the data processing, which is carried out for calculating the magnetization direction of the permanent magnet 21, becomes easy to perform. Accordingly, the responsiveness of the calculation for the magnetization direction of the permanent magnet 21 becomes better, and the controllability in the generation direction of the magnetic gradient and the controllability in the rotation correction of the acquired image data are improved.

When the magnetic field intensity of the direction detection magnetic field $M_1$ is made lower than the magnetic field intensity of the guidance magnetic field, the direction of the capsule medical device 403 does not change and thereby the X-axis direction of the capsule medical device 403 can be calculated more accurately.

Fig. 39 is a schematic diagram illustrating another example of a capsule medical device applied to the second embodiment of the present invention. Fig. 40 is a front view illustrating the configuration of the capsule medical device of Fig. 39.

Note that, while the above second embodiment to the third modification of the second embodiment are described as applied to an example in which the center axis line of the oscillation coil is disposed substantially parallel to the magnetization direction of the permanent magnet, the center axis line of the oscillation coil may be disposed significantly away from the magnetization direction (e.g., substantially perpendicular), as shown in Fig. 39 and Fig. 40; the arrangement thereof is not particularly limited.

By disposing the oscillation coil in this manner, when the shift between the magnetization direction of the permanent magnet and the magnetization direction of guidance magnetic field is small, a direction substantially perpendicular to a plane including the center axis line of the oscillation coil and the magnetization direction of the guidance magnetic field is calculated as the f-axis direction of the capsule medical device.

When the shift between the magnetization direction of the permanent magnet and the magnetization direction of the guidance magnetic field is large, the f-axis direction of the capsule medical device is preferably calculated by the same method as in the above second embodiment to the third modification of the second embodiment.

[Third embodiment]

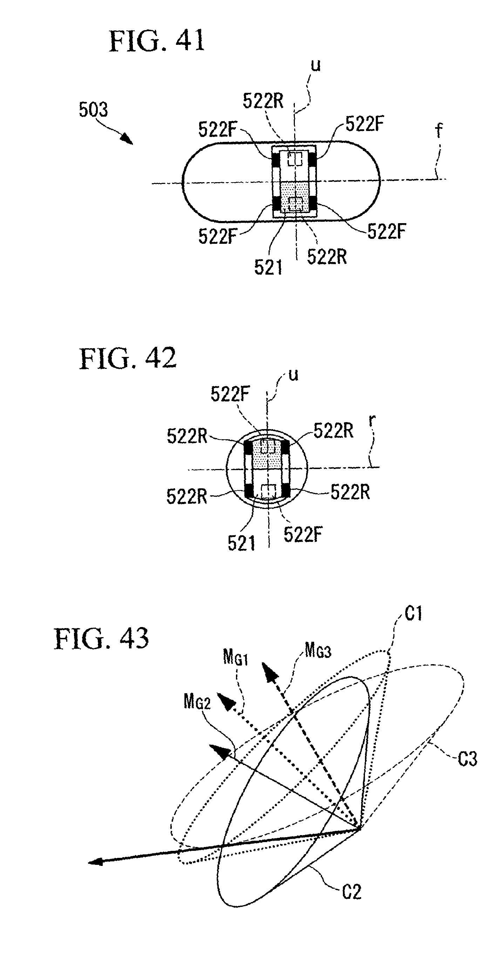

Next, a third embodiment of the present invention will be described with reference to Fig. 41 to Fig. 43.

While the basic configuration of a medical device guidance system in the present embodiment is the same as that in the first embodiment, a method of detecting the f-axis direction of the capsule medical device is different from that in the first embodiment. Accordingly, in the present embodiment, only the method of detecting the f-axis direction of the capsule medical device will be described by use of Fig. 41 to Fig. 43, and a description of the medical device guidance system configuration etc. will be omitted.

Fig. 41 is a vertical cross-sectional view illustrating the configuration of a permanent magnet and a surrounding part thereof in a capsule medical device in the present embodiment.

Fig. 42 is a front view illustrating the configuration of the permanent magnet and the surrounding part thereof in the capsule medical device of Fig. 41.

Note that the same constituent parts as those in the first embodiment are denoted by the same symbols, and a description thereof will be omitted.

A capsule medical device (medical device) 503 of a medical device guidance system 501 is provided with a permanent magnet (magnetic field response part) 521, which has a magnetization direction corresponding to a radial direction of the capsule medical device 503, and force sensors (magnetic force measurement parts) 522R and 522F detecting a rotational torque applied to the permanent magnet 521, as shown in Fig. 41 and Fig. 42.

Note that the medical device guidance system 501 is not provided with the position and orientation detection part 33.

The permanent magnet 521 has a pair of faces formed substantially perpendicular to the f-axis of the capsule medical device and a pair of faces formed substantially perpendicular to the r-axis of the capsule medical device.

On the pair of faces substantially perpendicular to the f-axis, at least four of the force sensors 522R are disposed for detecting a rotational torque applied to the permanent magnet 521 around the r-axis (axis in a direction perpendicular to the plane of the paper in Fig. 41). On the pair of faces substantially perpendicular to the r-axis, at least four of the force sensors 522F are disposed for detecting a rotational torque applied to the permanent magnet 521 around the f-axis (axis in a direction perpendicular to the plane of the paper in Fig. 42).

Here, the method of detecting the f-axis direction in the capsule medical device 503 will be described as a feature of the present modification. Note that the outlines of a method of guiding the capsule medical device 503 and a method of acquiring the image are the same as those in the first embodiment, and a description thereof will be omitted.

Fig. 43 is a vector diagram illustrating a position detection magnetic field applied to the capsule medical device of Fig. 41.

First, a position detection magnetic field $M_{G1}$ having any magnetic field direction is applied to the permanent magnet 521 of the capsule medical device 503, as shown in Fig. 43.

When the position detection magnetic field $M_{G1}$ is applied to the permanent magnet 521, a rotational torque is applied to the permanent magnet 521 and presses the force sensors 522R and 522F. The pressure force is detected by the force sensors 522R and 522F, and the detection signals of the force sensors 522R and 522F are superimposed on the image data and transmitted to the image data receiving part 43 of the external device.

The rotational torque T applied to the permanent magnet 521 is obtained on the basis of the detection signals of the force sensors 522R and 522F and the arrangement of the force sensors 522R and 522F. Meanwhile, the rotational torque T applied to the permanent magnet 521 is expressed by the following formula (11) using the magnetic field intensity H of the position detection magnetic field $M_{G1}$, the magnetic field intensity M of the permanent magnet 521, and an angle $\theta_{G1}$ formed by the magnetization direction of the permanent magnet 521 and the magnetic field direction of the position detection magnetic field $M_{G1}$.

$$T = HM \cdot \cos \theta_{G1} \quad \cdots \quad (11)$$

By solving this formula (11), the angle $\theta_{G1}$ formed by the magnetization direction of the permanent magnet 521 and the magnetic field direction of the position detection magnetic field $M_{G1}$ is obtained. From this formed angle $\theta_{G1}$ is obtained a conical plane C1 having the center axis line in the magnetic field direction of the position detection magnetic field $M_{G1}$, which is possibly the magnetization direction of the permanent magnet 521.

Subsequently, a position detection magnetic field $M_{G2}$ and a position detection magnetic field $M_{G3}$, which have magnetization directions different from the magnetic field direction of the position detection magnetic field $M_{G1}$, are applied to the permanent magnet 521, and a conical plane C2 having the center axis line in the magnetic field direction of the position detection magnetic field $M_{G2}$ and a conical plane C3 having the center axis line in the magnetic field direction of the position detection magnetic field $M_{G3}$ are similarly obtained.

From the conical planes C1 to C3 obtained in this manner, a common intersection line among these planes is calculated as the magnetization direction of the permanent magnet 521 in the capsule medical device 503.

Further, since the f-axis direction of the capsule medical device 503 has a relationship of 90° - θ with respect to the calculated magnetization direction of the permanent magnet 521, the f-axis direction of the capsule medical device 503 is also calculated.

By the above configuration, it is possible to calculate the f-axis direction of the capsule medical device 503 by calculating angles $\theta_{G1}$, $\theta_{G2}$, and $\theta_{G3}$ formed by the magnetization directions of the direction detection magnetic fields $M_{G1}$, $M_{G2}$, and $M_{G3}$ and the magnetization direction of the permanent magnet 521, respectively, according to the detection signals of the force sensors 522R and 522F.

Since the force sensors 522R and 522F directly measure the rotational torque generated in the permanent magnet 521, the formed angles $\theta_{G1}$, $\theta_{G2}$, and $\theta_{G3}$ are calculated accurately.

The data processing calculating the formed angles $\theta_{G1}$, $\theta_{G2}$, and $\theta_{G3}$ becomes easy to perform because the position detection calculation and the image processing are not necessary. Accordingly, the responsiveness of the calculation for the magnetization direction of the permanent magnet 521 becomes better, and the controllability in the generation direction of the magnetic gradient and the controllability in the rotation correction of the acquired image data are improved.

When the magnetic field intensities of the direction detection magnetic fields $M_{G1}$, $M_{G2}$, and $M_{G3}$ are made lower than the magnetic field intensity of the guidance magnetic field, the direction of the capsule medical device 503 does not change and thereby the f-axis direction of the capsule medical device 503 can be calculated more accurately.

[Fourth embodiment]

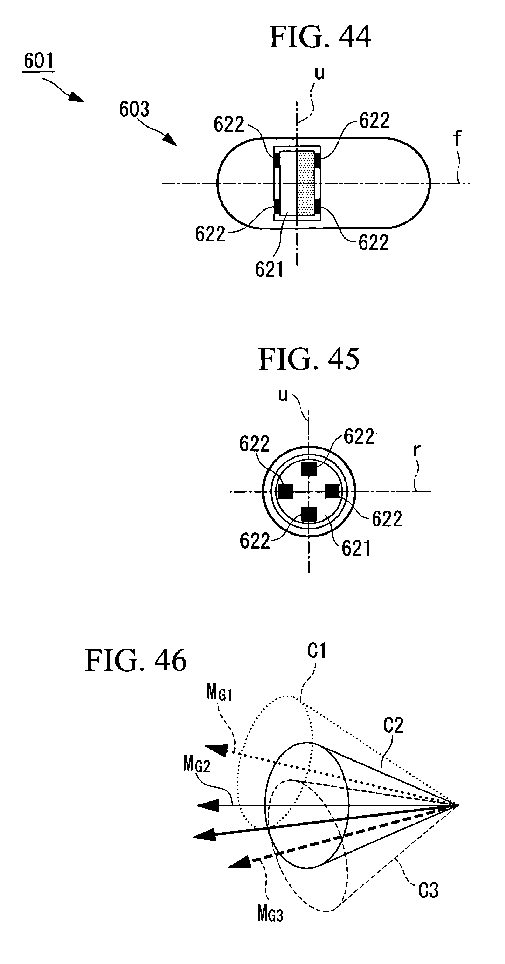

Next, a fourth embodiment of the present invention will be described with reference to Fig. 44 to Fig. 48.

While the basic configuration of a medical device guidance system in the present embodiment is the same as that in the first embodiment, a method of detecting the f-axis direction of the capsule medical device is different from that in the first embodiment. Accordingly, in the present embodiment, only the method of detecting the f-axis direction of the capsule medical device will be described by use of Fig. 44 to Fig. 48, and a description of the medical device guidance system configuration etc. will be omitted.

Fig. 44 is a vertical cross-sectional view illustrating the configuration of a permanent magnet and a surrounding part thereof in a capsule medical device in the present embodiment.

Fig. 45 is a front view illustrating the configuration of the permanent magnet and the surrounding part thereof in the capsule medical device of Fig. 44.

Note that the same constituent parts as those in the first embodiment are denoted by the same symbols, and a description thereof will be omitted.

A capsule medical device (medical device) 603 of a medical device guidance system 601 is provided with a permanent magnet (magnetic field response part) 621, which has a magnetization direction corresponding to a radial direction of the capsule medical device 603, and a force sensor (magnetic force measurement part) 622 detecting a rotational torque applied to the permanent magnet 621, as shown in Fig. 44 and Fig. 45.

Note that the medical device guidance system 601 is not provided with the position and orientation detection part 33.

The permanent magnet 621 has a pair of faces formed substantially perpendicular to the f-axis of the capsule medical device.

On the pair of faces substantially perpendicular to the f-axis, at least eight of the force sensors 622 are disposed for detecting rotational torques applied to the permanent magnet 621 around the r-axis and the u-axis.

Fig. 46 is a vector diagram illustrating a position detection magnetic field applied to the capsule medical device of Fig. 44.

Here, the method of detecting the f-axis direction in the capsule medical device 603 will be described as a feature of the present modification.

First, a position detection magnetic field $M_{G1}$ having any magnetic field direction is applied to the permanent magnet 621 of the capsule medical device 603, as shown in Fig. 46.

When the position detection magnetic field $M_{G1}$ is applied to the permanent magnet 621, a rotational torque is applied to the permanent magnet 621 and presses the force sensors 622. The pressure force is detected by the force sensors 622, and a detection signal of the force sensors 622 is superimposed on the image data and transmitted to the image data receiving part 43 of the external device.

After that, as in the third embodiment, a common intersection line of a conical plane C1 having the center axis line in the magnetic field direction of the position detection magnetic field $M_{G1}$, a conical plane C2 having the center axis line in the magnetic field direction of the position detection magnetic field $M_{G2}$, and a conical plane C3 having the center axis line in the magnetic field direction of the position detection magnetic field $M_{G3}$ is calculated as the f-axis direction of the capsule medical device 603.

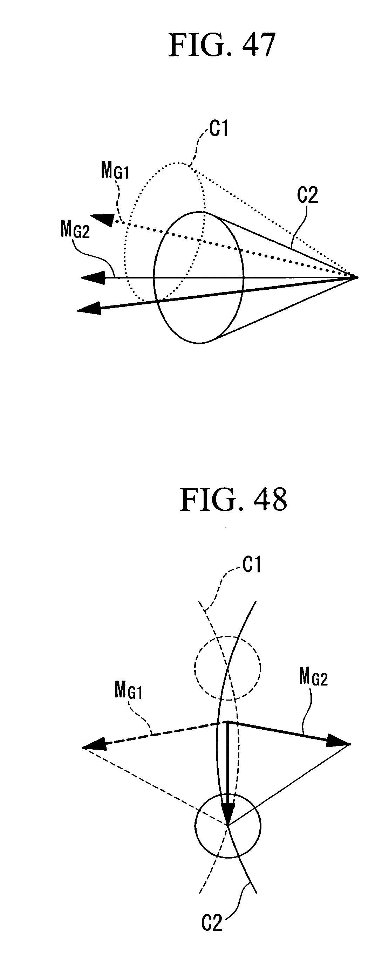

Fig. 47 is a vector diagram illustrating another application example of the position detection magnetic field applied to the capsule medical device of Fig. 44. Fig. 48 is a diagram illustrating the method of determining the f-axis direction of the capsule medical device of Fig. 47.

Note that the f-axis direction of the capsule medical device 603 may be calculated by use of the position detection magnetic fields $M_{G1}$, $M_{G2}$, and $M_{G3}$, as described above, or the f-axis direction of the capsule medical device 603 may be calculated by use of only the position detection magnetic fields $M_{G1}$ and $M_{G2}$, as shown in Fig. 47; the calculation method is not particularly limited.

At this time, two sets of the f-axis direction of the capsule medical device 603 are obtained, as shown in Fig. 48. Accordingly, a direction in which a positional relationship between the position detection magnetic fields $M_{G1}$ and $M_{G2}$ and the f-axis direction of the capsule medical device corresponds to the direction of the force applied to the permanent magnet by the position detection magnetic fields $M_{G1}$ and $M_{G2}$ is determined as the f-axis direction of the capsule medical device 603.

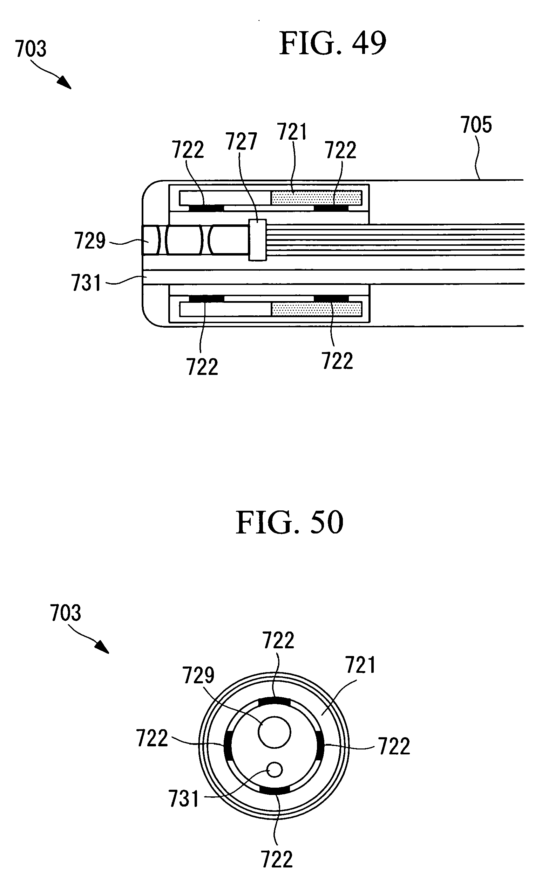

Fig. 49 is a diagram illustrating a case where, instead of the capsule medical device, an endoscope device is used as the medical device applied to the medical device guidance system in the present embodiment. Fig. 50 is a front view illustrating the configuration of the endoscope device of Fig. 49.

Note that, while the above described fourth embodiment has been described using the capsule medical device as the medical device, an endoscope device 703 may be used, as shown in Fig. 49 and Fig. 50; the medical device is not particularly limited.

The endoscope device (medical device) 703 is provided with an endoscope 705 inserted into a body cavity of a subject and a permanent magnet (magnetic field response part) 721 guiding the front end of the endoscope 705, as shown in Fig. 49 and Fig. 50.

The endoscope 705 is provided with an imaging sensor 727 imaging the inside of the body cavity, a lens group 729 forming an image of the body cavity onto the imaging sensor 727, and a forceps hole 731 guiding forceps to the front end of the endoscope 705.

The permanent magnet 721 is formed in a cylindrical shape in which the imaging sensor 727, the lens group 729, and the forceps 731 are inserted, and is magnetized in the f-axis direction (left-right direction in Fig. 49). Between the permanent magnet 721 and the endoscope 705, a force sensor (magnetic force measurement part) 722 is disposed for detecting a rotational torque applied to the permanent magnet 721.

The force sensor 722 is a sensor detecting the rotational torque rotating the permanent magnet 721 around the r-axis and the u-axis, and specifically detects a force pressing the force sensor 722 when the permanent magnet 721 rotates.

A method of calculating the f-axis direction of the endoscope device 703 having such a configuration is the same as that in the above fourth embodiment and a description thereof will be omitted.

**Claims**

1.   A medical device position detection system, comprising:

   a medical device introduced into a subject's body;
   a magnetic field response part that is disposed in the medical device, responds to a magnetic field by virtue of possessing a magnetization direction, and guides the medical device;
   a magnetic field generation part generating a magnetic field within the subject's body;
   a direction detection magnetic field control part generating a direction detection magnetic field, from the magnetic field generation part, for detecting a direction of the medical device;
   a response detection part detecting the response of the magnetic field response part due to the direction detection magnetic field; and
   a direction calculation part calculating the direction of the medical device according to a direction of the direction detection magnetic field and a detection result of the response detection part.

2.   The medical device position detection system according to Claim 1, wherein
   directions of two axes having different directions from each other among three axis directions having different directions from one another in the medical device, are calculated by the response detection part, and a direction of an axis intersecting a plane formed by the two axes is calculated by the direction calculation part.

3.   The medical device position detection system according to Claim 1 or 2, wherein
   the response detection part includes an image acquisition part acquiring an image of inside the subject's body.

4.   The medical device position detection system according to Claim 1 or 2, wherein
   the response detection part includes a magnetic force measurement part measuring a force generated in the magnetic field response part.

5.   The medical device position detection system according to any of Claims 1 to 4, wherein
   the direction detection magnetic field control part controls the magnetic field generation part to generate a static magnetic field.

6.   The medical device position detection system according to Claim 5, wherein
   the direction detection magnetic field control part controls the magnetic field generation part to sequentially generate a plurality of magnetic fields having different directions or intensities from one another, and
   the direction calculation part calculates the direction of the medical device according to respective detection results of the response detection part for the plurality of magnetic fields.

7.   The medical device position detection system according to any of Claims 1 to 4, wherein
   the direction detection magnetic field control part controls the magnetic field generation part to generate a gradient magnetic field.

8.   The medical device position detection system according to Claim 2, wherein
   the medical device has a substantially cylindrical shape,
   the magnetization direction of the magnetic field response part is substantially perpendicular to a center axis of the substantially cylindrical shape, and
   the plane formed by the two axes detected by the response detection part is substantially parallel to the center axis.

9.   The medical device position detection system according to Claim 2, wherein
   the medical device has a substantially cylindrical shape,
   the magnetization direction of the magnetic field response part is substantially perpendicular to a center axis of the substantially cylindrical shape, and
   the plane formed by the two axes detected by the response detection part is substantially perpendicular to the center axis.

**10.** The medical device position detection system according to Claim 4, wherein
the magnetic force measurement part is a sensor measuring at least one of pressure, distortion, and torque applied to the magnetic field response part, and
the magnetic field response part is fixed to the medical device via the sensor.

**11.** A medical device guidance system, comprising:

a medical device position detection system according to any one of Claims 1 to 10; and
a guidance magnetic field control part generating a guidance magnetic field from the magnetic field generation part for guiding the medical device, wherein
the guidance magnetic field control part controls the magnetic field generation part according to a calculation result in the direction calculation part.

**12.** The medical device guidance system according to Claim 11, wherein
the intensity of the magnetic field generated from the magnetic field generation part by the direction detection magnetic field control part is lower than the intensity of the magnetic field generated from the magnetic field generation part by the guidance magnetic field control part.

**13.** A position detection method of a medical device guidance system, comprising the steps of:

a magnetic field generation part generating a direction detection magnetic field for detecting a magnetization direction of a magnetic field response part provided in a medical device;
detecting a response of the magnetic field response part; and
detecting a direction of the medical device from the response of the magnetic field response part and a direction of the direction detection magnetic field.

**14.** A guidance method of a medical device guidance system, wherein after the step of detecting a direction in a position detection method of the medical device guidance system according to Claim 13, the guidance method comprises the step of
the magnetic field generation part generating a guidance magnetic field, for guiding the medical device, according to the detected direction of the medical device.

# FIG. 1

# FIG. 2

# FIG. 3

# FIG. 4

# FIG. 5

FIG. 6

## FIG. 7

## FIG. 8

## FIG. 9

# FIG. 10

57

P

# FIG. 11

P

57

α

# FIG. 12

H

θ

M

$T = MH \, sin \, \theta$

## FIG. 13

M2

21

## FIG. 14

3

θ3

M2

21

## FIG. 15

3

Z

M R1

Y

X

# FIG. 16

# FIG. 17

# FIG. 18

FIG. 19

FIG. 20A

FIG. 20B

FIG. 21

# FIG. 22

# FIG. 23

# FIG. 24

# FIG. 25

## FIG. 26

## FIG. 27

## FIG. 28

## FIG. 29

## FIG. 30

## FIG. 31

57

P

## FIG. 32

P2

57

M$_D$    M$_R$

P1

## FIG. 33

P2

57

$\alpha$

P1

# FIG. 34

# FIG. 35

# FIG. 36

# FIG. 37

# FIG. 38

Z

M₁

α

X-Y

# FIG. 39

u

315

f

21

# FIG. 40

u

315

r

## FIG. 41

503

522R
u
522F          522F
f
522F          522F
521     522R

## FIG. 42

u
522F
522R          522R
r
522R          522R
521     522F

## FIG. 43

$M_{G1}$   $M_{G3}$   C1

$M_{G2}$

C3

C2

## FIG. 44

## FIG. 45

## FIG. 46

# FIG. 47

# FIG. 48

## FIG. 49

## FIG. 50

| **INTERNATIONAL SEARCH REPORT** | International application No. |
| --- | --- |
| | PCT/JP2007/071873 |

A. CLASSIFICATION OF SUBJECT MATTER
*A61B1/00*(2006.01)i, *A61B5/06*(2006.01)i, *A61B5/07*(2006.01)i, *A61B19/00*
(2006.01)i, *A61J3/07*(2006.01)n

According to International Patent Classification (IPC) or to both national classification and IPC

B. FIELDS SEARCHED

Minimum documentation searched (classification system followed by classification symbols)
A61B1/00, A61B5/06, A61B5/07, A61B19/00, A61J3/07

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2003-299612 A (Olympus Optical Co., Ltd.), 21 October, 2003 (21.10.03), Par. Nos. [0001] to [0038] & US 2003/0229268 A1 | 1,3 |
| A | JP 2006-149668 A (Olympus Corp.), 15 June, 2006 (15.06.06), Full text; all drawings & WO 2006/057443 A1 | 2,4-12 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 December, 2007 (05.12.07) | 04 March, 2008 (04.03.08) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**INTERNATIONAL SEARCH REPORT**

International application No.

PCT/JP2007/071873

| Box No. II Observations where certain claims were found unsearchable (Continuation of item 2 of first sheet) |
|---|

This international search report has not been established in respect of certain claims under Article 17(2)(a) for the following reasons:

1. ☒ Claims Nos.: 13 and 14
because they relate to subject matter not required to be searched by this Authority, namely:
  Claims 13 and 14 relate to preliminary treatment methods for measuring conditions inside the human body for medical purpose and thus relate to a subject matter which this International Search Authority is not required,
  (continued to extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III Observations where unity of invention is lacking (Continuation of item 3 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| **INTERNATIONAL SEARCH REPORT** | International application No. |
| --- | --- |
| | PCT/JP2007/071873 |

Continuation of Box No.II-1 of continuation of first sheet(2)

under the provisions of Article 17 (2)(a)(i) of PCT and Rule 39.1 (iv) of the Regulations under PCT, to search.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003299612 A
- JP 2005103091 A